# EUROPÄISCHE PATENTSCHRIFT

(11) **EP 1 201 675 B1**
(45) Veröffentlichungstag und Bekanntmachung des Hinweises auf die Patenterteilung: **21.01.2004**
(21) Anmeldenummer: 01122420.1
(22) Anmeldetag: 20.09.2001
(51) Int. Cl.: C07F 9/6574

(54) **Bisphosphitverbindungen und deren Metallkomplexe**
Bisphosphites and Metal Complexes thereof
Bisphosphites et leurs complexes de métaux

(30) Priorität: 27.10.2000 DE 10053272
(43) Veröffentlichungstag der Anmeldung: 02.05.2002
(73) Patentinhaber: Oxeno Olefinchemie GmbH, 45772 Marl (DE)
(72) Erfinder: Röttger, Dirk, Dr., 45657 Recklinghausen (DE); Hess, Dieter, Dr., 45770 Marl (DE); Wiese, Klaus-Diether, Dr., 45721 Haltern (DE); Borgmann, Cornelia, Dr., 45657 Recklinghausen (DE); Börner, Armin, Prof. Dr., 18059 Rostock (DE); Selent, Detlef, Dr., 10318 Berlin (DE); Schmutzler, Reinhard, Prof. Dr., 38304 Wolfenbüttel (DE); Kunze, Christine, 38533 Vordorf (DE)

(56) Entgegenhaltungen:
- DE-A- 19 602 301
- SELENT, DETLEF ET AL: "New phosphorus ligands for the rhodium-catalyzed isomerization/ hydroformylation of internal octenes" ANGEW. CHEM., INT. ED. (2001), 40(9), 1696-1698 , XP002186807
- KADYROV R ET AL: "New carbohydrate bisphosphites as chiral ligands" TETRAHEDRON: ASYMMETRY, ELSEVIER SCIENCE PUBLISHERS, AMSTERDAM, NL, Bd. 9, Nr. 2, 30. Januar 1998 (1998-01-30), Seiten 329-340, XP004131180 ISSN: 0957-4166
- SHADID B; VAN DER PLAS H C: "The Synthesis of Cytokinin Phophatases" TETRAHEDRON ELSEVIER SCIENCE PUBLISHERS, Bd. 46, Nr. 3, 1990, Seiten 901-912, XP002186808 AMSTERDAM, NL
- SIEDENTOP T; NEDA I; SCHMUTZLER R.; "SYNTHESE UND KOMPLEXIERUNG PHOSPHORYLIERTER SPACER-MODIFIZIERTER GLUCOSEDERIVATE"; ZEITSCHRIFT FUR NATURFORSCHUNG, TEIL B: ANORGANISCHE CHEMIE, ORGANISCHE CHEMIE, VERLAG DER ZEITSCHRIFT FUR NATURFORSCHUNG; TUBINGEN, DE; VOL - 55; NR. - 10 Seite

## Beschreibung

Die vorliegende Erfindung betrifft Bisphosphite und deren Metallkomplexe, die Herstellung, sowie die Verwendung der Bisphosphite als Liganden in katalytischen Reaktionen.

Die Reaktionen zwischen Olefinverbindungen, Kohlenmonoxid und Wasserstoff in Gegenwart eines Katalysators zu den um ein C-Atom reicheren Aldehyden ist als Hydroformylierung (Oxierung) bekannt. Als Katalysatoren in diesen Reaktionen werden häufig Verbindungen der Übergangsmetalle der VIII. Gruppe des Periodensystems der Elemente verwendet, insbesondere Verbindungen des Rhodiums und des Kobalts. Die Hydroformylierung mit Rhodiumverbindungen bietet im Vergleich zur Katalyse mit Kobaltverbindungen in der Regel den Vorteil höherer Selektivität und ist damit meistens wirtschaftlicher. Bei der durch Rhodium katalysierten Hydroformylierung werden zumeist Komplexe eingesetzt, die aus Rhodium und bevorzugt aus trivalenten Phosphorverbindungen als Liganden bestehen. Bekannte Liganden sind beispielsweise Verbindungen aus den Klassen der Phosphine, Phosphite und Phosphonite. Eine gute Übersicht über den Stand der Hydroformylierung von Olefinen findet sich in B. CORNILS, W. A. HERRMANN, *"Applied Homogeneous Catalysis with Organometallic Compounds"*, Vol. 1&2, VCH, Weinheim, New York, 1996.

Jedes Katalysatorsystem (Kobalt oder Rhodium) hat seine spezifischen Vorzüge. Je nach Einsatzstoff und Zielprodukt kommen daher unterschiedliche Katalysatorsysteme zum Einsatz, wie folgende Beispiele zeigen. Arbeitet man mit Rhodium und Triphenylphosphin, lassen sich α-Olefine bei niedrigeren Drücken hydroformylieren. Als Phosphor-haltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

Die Patente US 4 694109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

Der Nachteil von zweizähnigen Phosphinliganden ist ein relativ hoher Aufwand, der zu ihrer Darstellung notwendig ist. Daher ist es oftmals nicht rentabel, solche Systeme in technischen Prozessen einzusetzen.

Siedentop, T.; Neda, I.; Schmutzler, R.; Z. Naturforschung, 55 b, Nr. 10, Seiten 956-960, Tübingen, 2000 beschreiben die Herstellung phosphorylierter spacermodifizierter Glucosederivate sowie die Darstellung entsprechender Platinkomplexe. Die Glucosederivate können als Phosphorhaltige Gruppierung unter anderem Naphto-1,3-dioxa-2-phophorin-4-on-Einheiten aufweisen. Ebenfalls wird dort die Verwendung von phosphorhaltigen Kohlenhydratderivaten in der asymmetrischen Hydrierung beschrieben.

Rhodium-Monophosphit-Komplexe sind geeignete Katalysatoren für die Hydroformylierung von verzweigten Olefinen mit innenständigen Doppelbindungen, jedoch ist die Selektivität für endständig hydroformylierte Verbindungen gering. Aus EP 0 155 508 ist die Verwendung von bisarylensubstituierten Monophosphiten bei der rhodiumkatalysierten Hydroformylierung von sterisch gehinderten Olefinen, z. B. Isobuten bekannt.

Rhodium-Bisphosphit-Komplexe katalysieren die Hydroformylierung von linearen Olefinen mit end- und innenständigen Doppelbindungen, wobei überwiegend endständig hydroformylierte Produkte entstehen, dagegen werden verzweigte Olefine mit innenständigen Doppelbindungen nur in geringem Maße umgesetzt.

Diese Phosphite ergeben bei ihrer Koordination an ein Übergangsmetallzentrum Katalysatoren von gesteigerter Aktivität, doch ist das Standzeitverhalten dieser Katalysatorsysteme, unter anderem wegen der Hydrolyseempfindlichkeit der Phosphitliganden, unbefriedigend. Durch den Einsatz von substituierten Bisaryldiolen als Edukte für die Phosphitliganden, wie in EP 0 214 622 oder EP 0 472 071 beschrieben, konnten erhebliche Verbesserungen erreicht werden.

Der Literatur zufolge sind die Rhodiumkomplexe dieser Liganden äußerst aktive Hydroformylierungskatalysatoren für α-Olefine. In den Patenten US 4 668 651, US 4 748 261 und US 4 885 401 werden Polyphosphitliganden beschrieben, mit denen α-Olefine, aber auch 2-Buten mit hoher Selektivität zu den terminal hydroformylierten Produkten umgesetzt werden können. Zweizähnige Liganden dieses Typs wurden auch zur Hydroformylierung von Butadien eingesetzt (US 5 312 996).

Obgleich die genannten Bisphosphite sehr gute Komplexliganden für Rhodium-Hydroformylierungskatalysatoren sind, ist es wünschenswert, deren Wirksamkeit noch weiter zu verbessern.

Es wurde gefunden, dass Bisphosphite der allgemeinen Struktur I 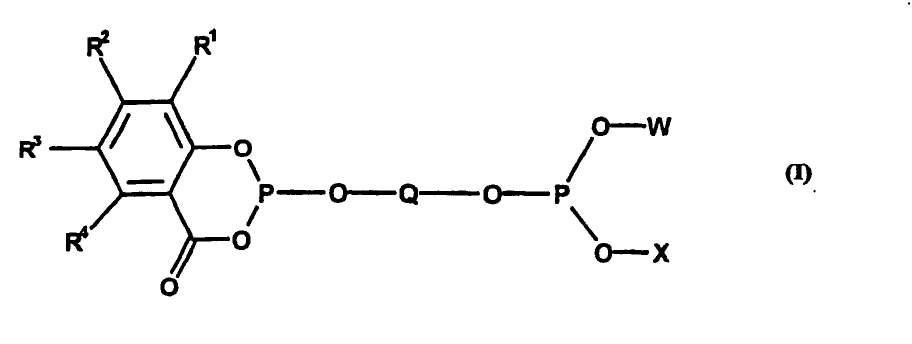 einfach hergestellt werden können und als Liganden bei Metall-katalysierten Reaktionen geeignet sind.

Gegenstand der vorliegenden Erfindung sind daher Bisphosphite der allgemeinen Formel I 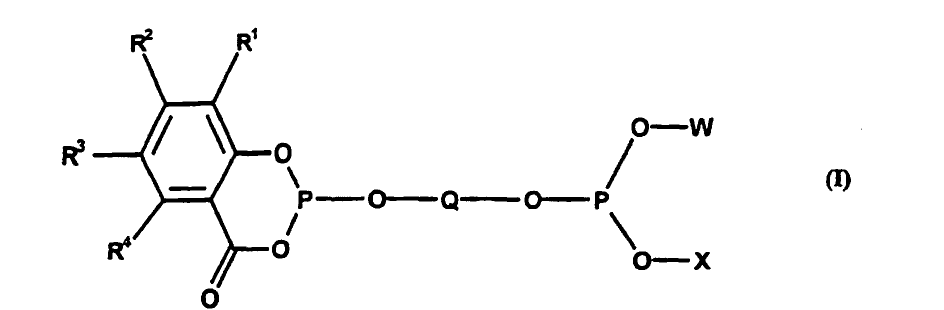 mit
R¹, R², R³, R⁴, = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR⁷, -COR⁷, -CO₂R⁷, -CO₂M, -SR⁷, - SO₂R⁷, -SOR⁷, -SO₃R⁷, -SO₃M, -SO₂NR⁷R⁸, NR⁷R⁸, N=CR⁷R⁸, NH₂, wobei R¹ bis R⁴ eine gleiche oder unterschiedliche Bedeutung besitzen und kovalent miteinander verknüpft sein können,
R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatisch-aromatischer, aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen, die gleich oder unterschiedlich oder kovalent miteinander verknüpft sein können.

Jeweils zwei der Reste R¹ bis R⁴ in Formel I können benzanneliert sein, d. h. jeweils R' und R², R² und R³ oder R³ und R⁴ können über einen aromatischen Ring miteinander verknüpft sein. Es sind somit drei Isomere realisierbar, die auch als Ligandensystem getrennt oder miteinander verwendet werden können. Die erfindungsgemäßen Bisphosphite der Formel I können daher auch gemäß den Formeln II, III und IV vorliegen.

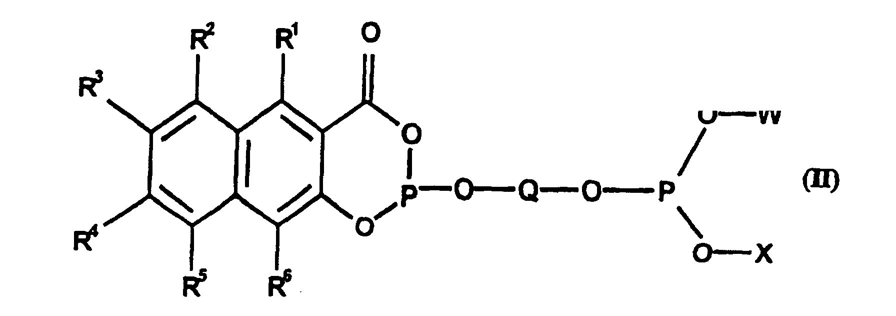

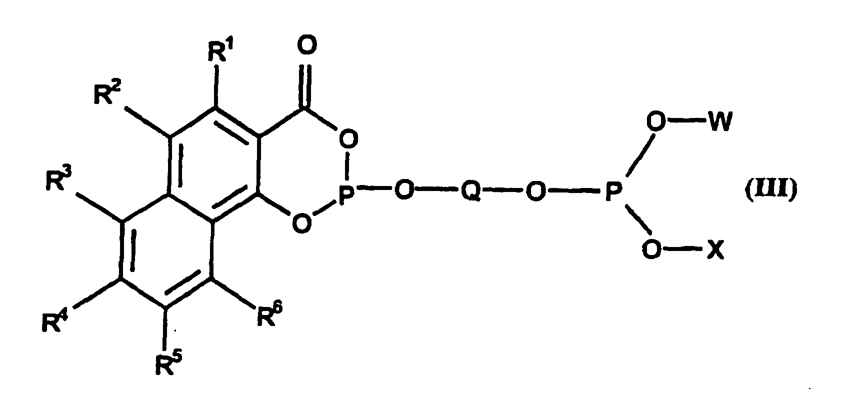

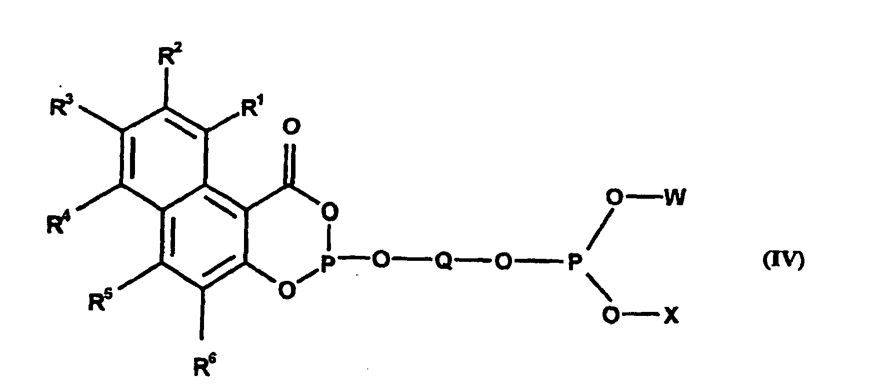

Die Bedeutungen der Reste R¹ bis R⁶ entsprechen denen der für Formel I definierten Bedeutungen für R¹ bis R⁴. Es ist möglich, dass diese Reste wiederum eine kovalente Verknüpfung miteinander aufweisen bzw. benzanneliert sind.

Spezielle Ausführungsformen der erfindungsgemäßen Bisphosphite betreffen Bisphosphite der Formeln V, VI und VII 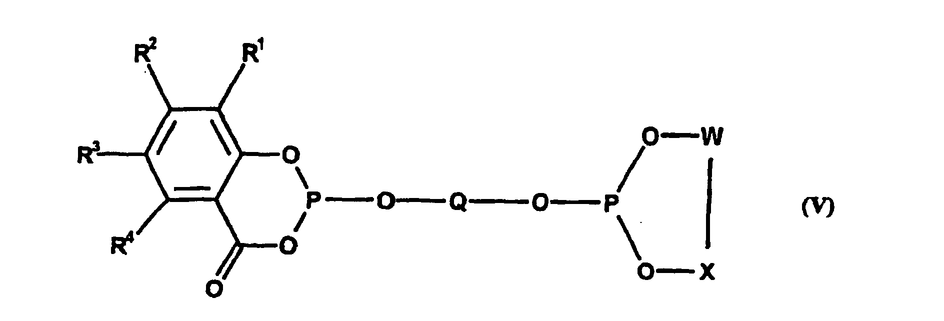 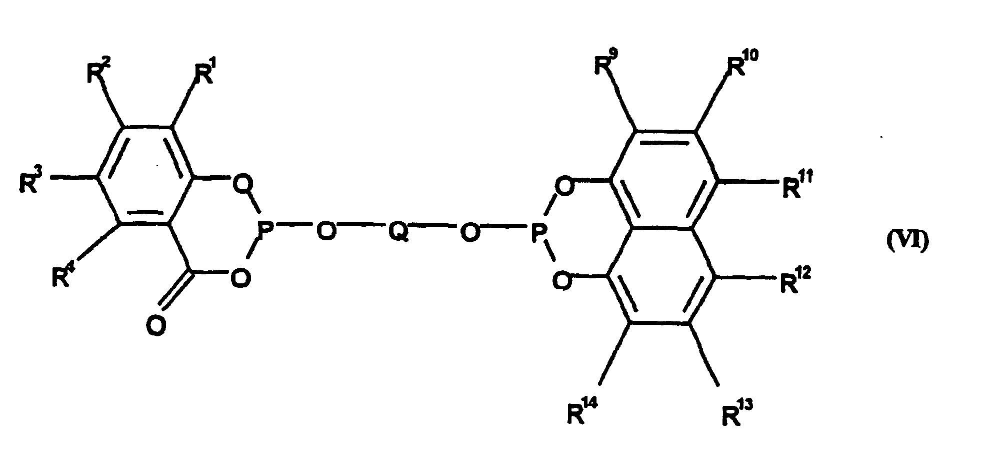 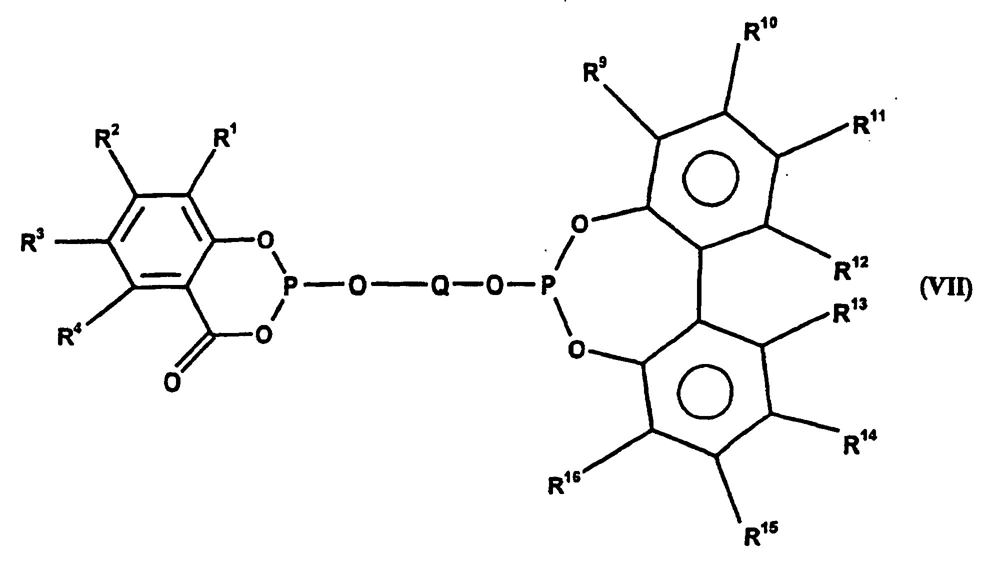 wobei W und X aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen bedeuten, X und W gleich oder unterschiedlich oder kovalent mit einander verknüpft sein können und R¹, R², R³, R⁴, R⁵, R⁶, R⁷, R⁸ und Q die bereits genannten Bedeutungen besitzen.
R⁹, R¹⁰, R¹¹, R¹², R¹³, R¹⁴, R¹⁵, R¹⁶ stehen für H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR²⁵, -COR²⁵, -CO₂R²⁵, -CO₂M, - SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R⁹ bis R¹⁶ eine gleiche oder unterschiedliche Bedeutung besitzen und kovalent miteinander verknüpft sein können.
M steht für ein Alkalimetall-, Erdalkalimetall-, Ammonium-, oder Phosphoniumion.

R²⁵ und R²⁶ können gleich oder unterschiedlich sein und jeweils für H, substituierte oder unsubstituierte, aliphatische oder aromatische Kohlenwasserstoffreste mit 1 bis 25 Kohlenstoffatomen, mit gleicher oder unterschiedlicher Bedeutung stehen.

Beispiele für Q sind bivalente Kohlenwasserstoffreste, die aliphatisch, alicyclisch, aliphatisch-alicyclisch, heterocyclisch, aliphatisch-heterocylisch, aromatisch, aromatisch-aromatisch oder aliphatisch-aromatisch sein können. Gegebenenfalls vorhandene Ringsysteme können ihrerseits mit den oben genannten Kohlenwasserstoffresten substituiert sein. In offenkettigen Strukturelementen können eine oder mehrere Methylengruppen durch Sauerstoff und/oder Schwefel und/oder NR¹ und/oder NH und/oder eine oder mehrere CH-Gruppen durch Stickstoff ersetzt sein.

Bevorzugt steht Q für bivalente. Reste, die aromatische Gruppen enthalten. Q kann beispielsweise ein Phenylenrest, Naphthylenrest, ein zweiwertiger Bisarylenrest oder ein bivalenter Rest eines Diphenylethers sein. Weiterhin kann Q die allgemeine Struktur -Ar-Z-Ar- haben. Darin bedeutet Ar einen mono- oder oligocyclischen bivalenten aromatischen Rest. Z steht entweder für eine direkte Bindung oder für eine gegebenenfalls substituierte Methylengruppe -CR²⁷R²⁸-, wobei R²⁷ und R²⁸ für Wasserstoff und/oder aliphatische und/oder aromatische Reste mit 1 bis 25 Kohlenstoffatomen stehen, die darüber hinaus Heteroatome enthalten können. Weiterhin können die Reste R²⁷ und R²⁸ zu einem oder mehreren Ringen verknüpft sein, d. h. eine kovalente Bindung aufweisen.

Von den Bisphosphiten nach den allgemeinen Formeln I, II, III, IV, V, VI und VII sind diejenigen besonders bevorzugt, bei denen der Rest Q für einen Kohlenwasserstoffrest (Bisarylenrest) nach der allgemeinen Formel VIII steht 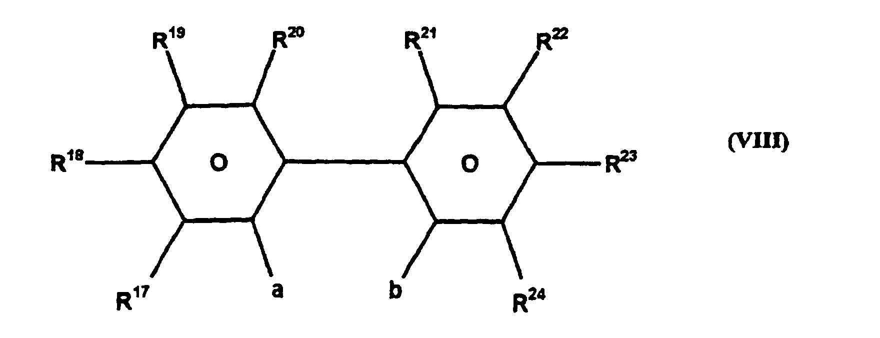 mit
R¹⁷, R¹⁸, R¹⁹, R²⁰, R²¹, R²², R²³, R²⁴ = H, aliphatischer, alicyclischer, aliphatisch-alicyclischer, heterocyclischer, aliphatisch-heterocyclischer, aromatisch-aromatischer, aromatischer, aliphatisch-aromatischer Kohlenwasserstoffrest mit 1 bis 50 Kohlenstoffatomen, F, Cl, Br, I, -CF₃, -OR²⁵, -COR²⁵, -CO₂R²⁵, -CO₂M, -SR²⁵, - SO₂R²⁵, -SOR²⁵, -SO₃R²⁵, -SO₃M, -SO₂NR²⁵R²⁶, NR²⁵R²⁶, N=CR²⁵R²⁶, NH₂, wobei R¹⁷ bis R²⁴ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion,
wobei die Positionen a und b als Anknüpfpunkte dieses Substituenten im Strukturelement O-Q-O in den Verbindungen der Formeln I bis VII stehen.

Gegenstand der vorliegenden Erfindung sind auch Bisphosphitmetallkomplexe, enthaltend ein Metall der 4., 5., 6., 7. oder 8. Nebengruppe des Periodensystems der Elemente und ein oder mehrere Bisphosphite der Formeln I, II, III, IV, V, VI und VII. Die Substituenten (R¹-R²⁶, Q, X, W) dieser Bisphosphite besitzen die bereits genannten Bedeutungen.

Im Folgenden werden repräsentative Beispiele von Liganden nach den allgemeinen Formeln I, II, III, IV, V, VI und VII im Sinne dieser Erfindung dargestellt, ohne den Schutzbereich der vorliegenden Erfindung zu beschränken.

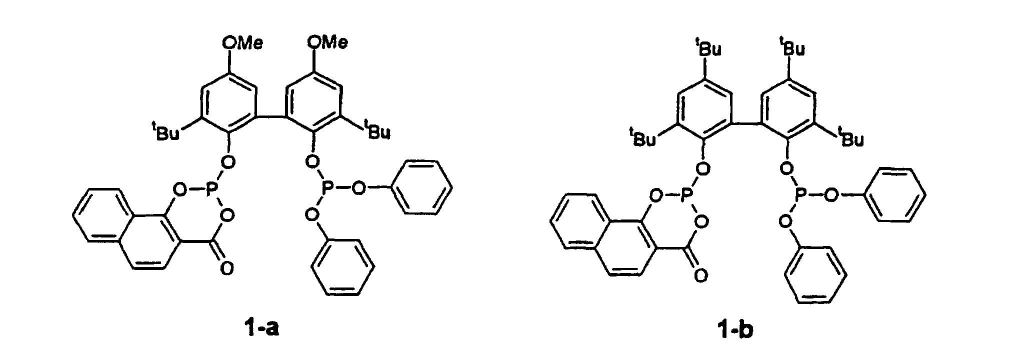

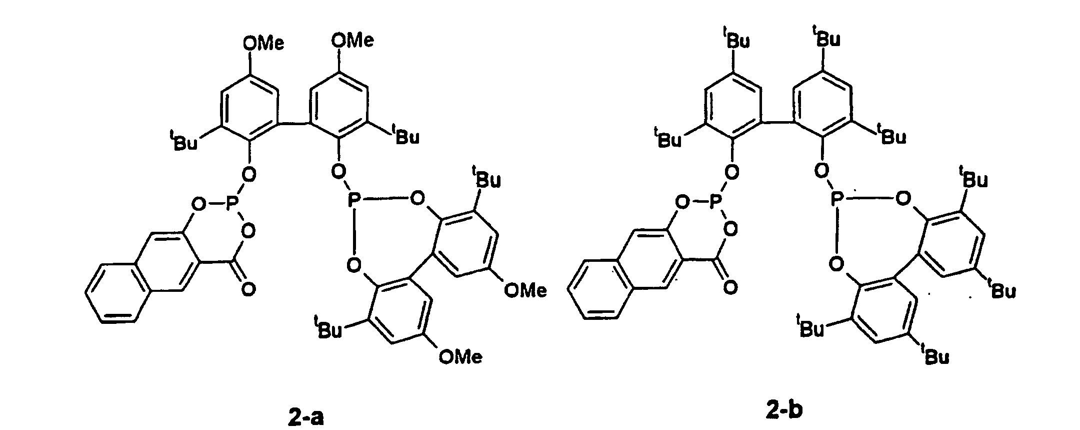

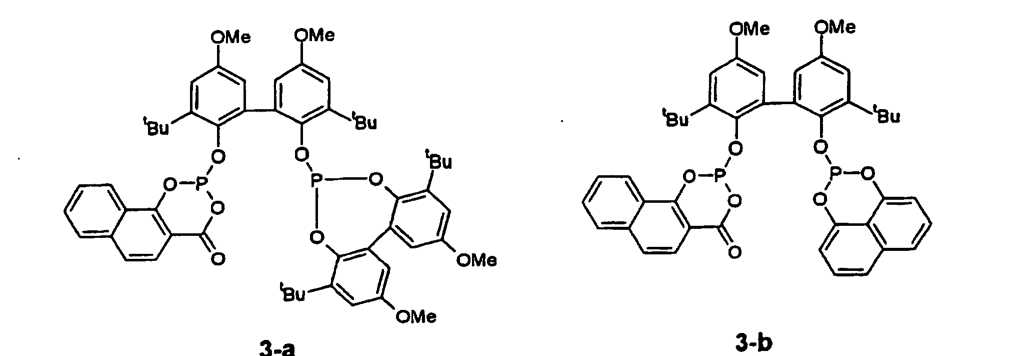

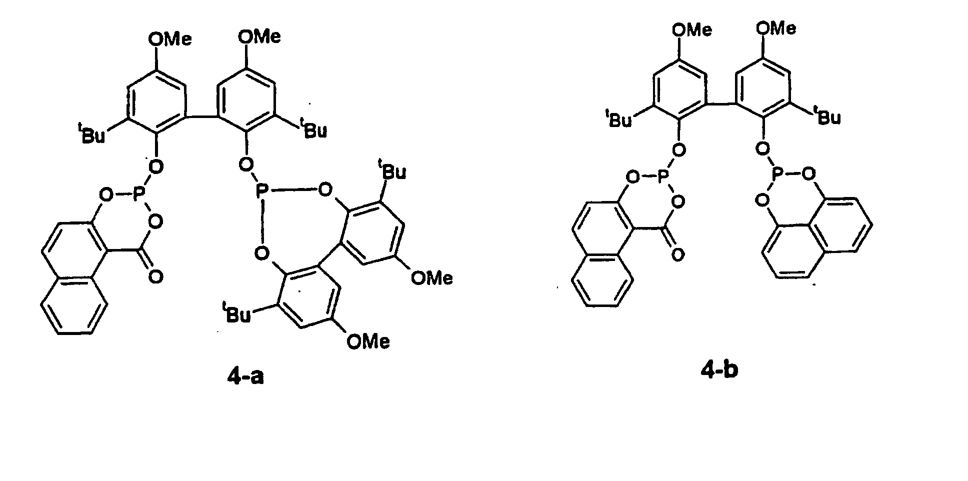

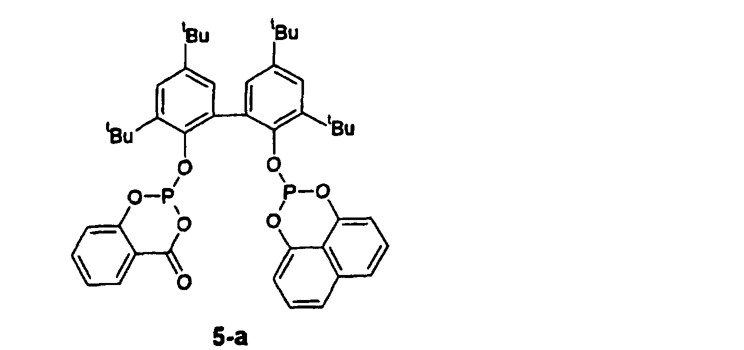

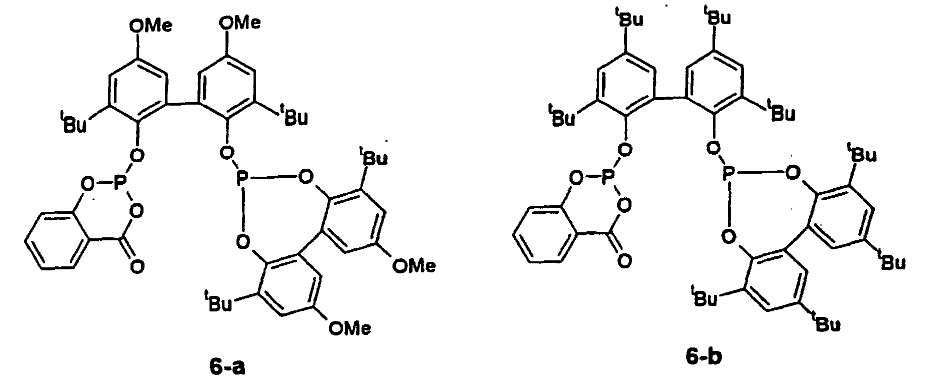

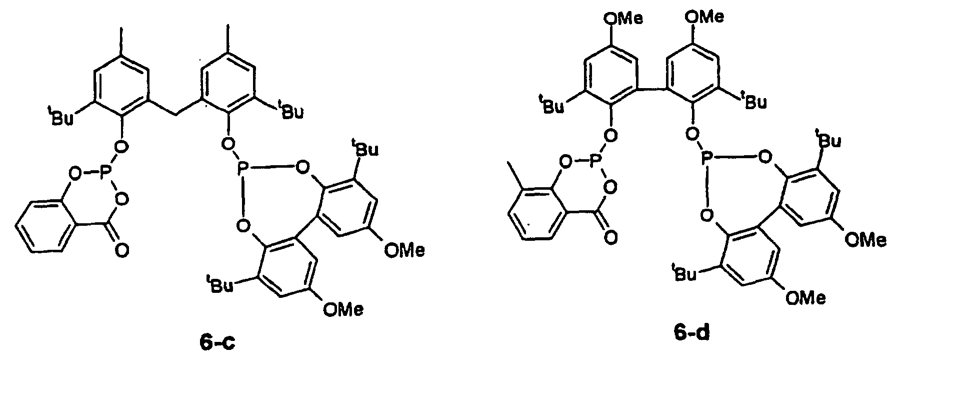

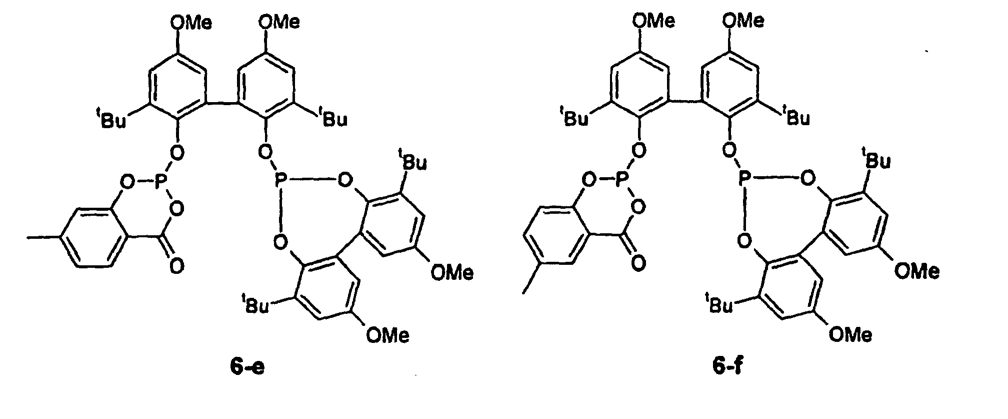

Die erfindungsgemäßen Bisphosphite können durch eine Folge von Reaktionen von Phosphorhalogeniden mit Alkoholen bzw. α-Hydroxyarylcarbonsäuren, bei denen Halogenatome am Phosphor gegen Sauerstoffgruppen ausgetauscht werden, hergestellt werden. Das grundsätzliche Vorgehen wird an einem Weg zu Verbindungen nach der allgemeinen Formel V illustriert
1) Eine α-Hydroxyarylcarbonsäure wird mit einem Phosphortrihalogenid, vorzugsweise Phosphortrichlorid in Gegenwart einer Base zu Zwischenprodukt A umgesetzt. 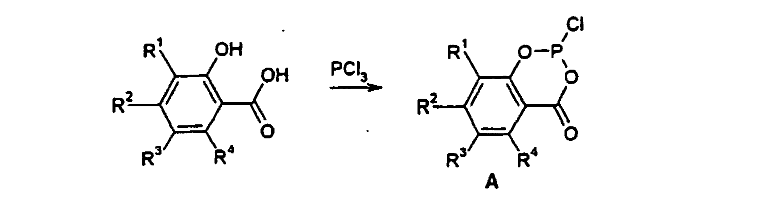
2) Ein Phosphortrihalogenid, vorzugsweise Phosphortrichlorid, wird mit einem Diol oder zwei Moläquivalenten Alkohol zu einem Monohalogenphosphit (Zwischenprodukt B) umgesetzt. 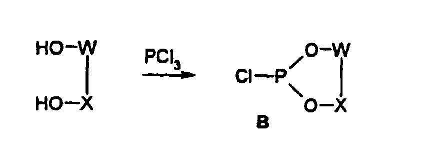
3) Aus dem Zwischenprodukt B wird durch Reaktion mit einem Diol (HO-Q-OH) ein hydroxyl-substituiertes Phosphit erhalten (Zwischenprodukt C). 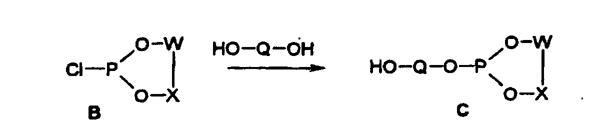
4) Aus der Reaktion von Zwischenprodukt A mit C wird das gewünschte Bisphosphit erhalten.

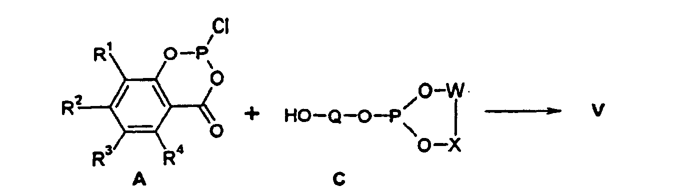

Dieser Syntheseweg ist nur einer von vielen, zeigt aber das grundsätzliche Vorgehen. Ein alternativer Weg ist zum Beispiel die Umsetzung von Zwischenprodukt A mit der Diolkomponente und anschließende Reaktion mit B zum Zielprodukt.

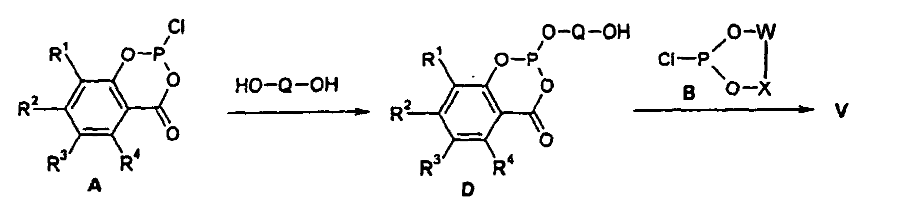

Da die eingesetzten Diole und ihre Folgeprodukte häufig fest sind, werden die Umsetzungen im Allgemeinen in Lösungsmitteln durchgeführt. Als Solventien werden nicht protische Lösungsmittel, die weder mit den Diolen noch mit den - Phosphorverbindungen reagieren, verwendet. Geeignete Lösungsmittel sind beispielsweise Tetrahydrofuran, Diethylether oder aromatische Kohlenwasserstoffe wie Toluol.

Bei der Umsetzung von Phosphorhalogeniden mit Alkoholen entsteht Halogenwasserstoff, der durch zugegebene Basen gebunden wird. Beispielsweise werden dafür tertiäre Amine, wie Triethylamin, eingesetzt. Teilweise ist es auch sinnvoll, die Alkohole vor der Reaktion in Metallalkoholate zu überführen, zum Beispiel durch Reaktion mit Natriumhydrid oder Butyllithium.

Die erfindungsgemäßen Bisphosphite der Formeln I, II, III, IV, V, VI und VII sind geeignete Bausteine für die Herstellung von Komplexen mit Metallen der 4., 5., 6., 7. oder 8. Nebengruppe des Periodensystems der Elemente. Insbesondere mit Metallen der 8. Nebengruppe können diese Komplexe als Katalysatoren für Carbonylierungsreaktionen oder Hydroformylierungsreaktionen verwendet werden, z. B. für die Hydroformylierung von C2-C25-Olefinen. Die Liganden zeichnen sich durch hohe Hydrolysestabilität aus. Besonders bei Einsatz von Rhodium als Katalysatormetall ergeben sich hohe katalytische Aktivitäten in Hydroformylierungsreaktionen. Aufgrund ihres hohen Molekulargewichtes besitzen die erfindungsgemäßen Bisphosphite eine geringe Flüchtigkeit. Sie können daher einfach von den leichter flüchtigen Reaktionsprodukten abgetrennt werden. Sie sind in den gängigen organischen Solventien ausreichend gut löslich.

Weitere Gegenstände der Erfindung sind die Verwendungen der Bisphosphite bzw. der Bisphosphitmetallkomplexe in Verfahren zur Hydroformylierung von Olefinen, bevorzugt mit 2 bis 25 Kohlenstoffatomen, zu den entsprechenden Aldehyden.

Zur Herstellung der katalytisch aktiven Metallkomplexe sind bevorzugt eingesetzte Metalle für die erfindungsgemäßen Bisphosphite Rhodium, Kobalt, Platin und Ruthenium. Aus den erfindungsgemäßen Liganden und dem Metall bildet sich unter Reaktionsbedingungen der aktive Katalysator. Die erfindungsgemäßen Liganden können dabei in freier Form in die Reaktionsmischung gegeben werden. Es ist weiterhin möglich, einen Übergangsmetallkomplex, der die o. g. Bisphosphitliganden enthält, als Precursor für den eigentlichen katalytisch aktiven Komplex einzusetzen. Der Hydroformylierungsprozess kann stöchiometrisch oder mit einer überschüssigen Menge an freien Bisphosphitliganden (z. B. 1 : 1 bis 1 : 200) durchgeführt werden.

Femer können auch Mischungen verschiedener Liganden - sowohl der erfindungsgemäßen Bisphosphite, hier auch die Isomeren gemäß den Formel II bis IV, als auch anderer geeigneter phosphorhaltiger Liganden als freie Ligandkomponente vorhanden sein.
Als zusätzliche, im Reaktionsgemisch vorhandene Liganden können Phosphine, Phosphite, Phosphonite oder Phosphinite eingesetzt werden.

Beispiele für solche Liganden sind:

Phosphine: Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin. Phosphite: Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2.4-di-t-butylphenyl)phosphit, Tris(2-t-butyl-4-methoxyphenyl)phosphit, Tris(2-t-butyl-4-methylphenyl)phosphit, Tris(p-kresyl)phosphit. Außerdem sind sterisch gehinderte Phosphitliganden, wie sie unter anderem in EP 155 508, US 4 668 651, US 4 748 261, US 4 769 498, US 4 774 361, US 4 835 299, US 4 885 401, US 5 059 710, US 5 113 022, US 5 179 055, US 5 260 491, US 5 264 616, US 5 288 918, US 5 360 938, EP 472 071, EP 518 241 und WO 97/20795 beschrieben werden, geeignete Liganden.

Phosphonite: Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind und Liganden, die in WO 98 43935, JP 09-268152 und DE 198 10 794 und in den deutschen Patentanmeldungen DE 199 54 721 und DE 199 54 510 beschrieben sind.

Gängige Phosphinitliganden sind unter anderem in US 5 710 344, WO 95 06627, US 5 360 938 oder JP 07082281 beschrieben. Beispiele hierfür sind Diphenyl(phenoxy)phosphin und dessen Derivate, in denen die Wasserstoffatome ganz oder teilweise durch Alkyl- und/oder Arylreste oder Halogenatome ersetzt sind, Diphenyl(methoxy)phosphin, Diphenyl(ethoxy)phosphin usw.
Im Allgemeinen werden bis 500, vorzugsweise 1 bis 200, bevorzugt 3 bis 50 Mol des erfindungsgemäßen Liganden pro Mol Gruppe-VIII-Übergangsmetall eingesetzt. Frischer Ligand kann zu jedem Zeitprodukt der Reaktion zugesetzt werden, um die Konzentration an freiem Liganden konstant zu halten. Die erfindungsgemäßen Übergangsmetall-Bisphosphitkomplex-Katalysatoren können vor ihrem Einsatz synthetisiert werden. In der Regel werden aber die katalytisch aktiven Komplexe aus einem Katalysatorvorläufer und dem erfindungsgemäßen Bisphosphitliganden in situ im Reaktionsmedium gebildet.

Als Katalysatorvorläufer kommen Salze oder Komplexe der Übergangsmetalle zum Einsatz. Beispiele sind Rhodiumcarbonyle, Rhodiumnitrat, Rhodiumchlorid, Rh(CO)₂(acac) (acac = Acetylacetonat), Rhodiumacetat, Rhodiumoctanoat oder Rhodiumnonanoat.

Die Konzentration des Metalls im Reaktionsgemisch liegt im Bereich von 1 ppm bis 1000 ppm, vorzugsweise im Bereich von 5 ppm bis 300 ppm.

Die mit den erfindungsgemäßen Bisphosphiten bzw. den entsprechenden Metallkomplexen durchgeführten Hydroformylierungsreaktionen erfolgten nach bekannten Vorschriften, wie z. B. in J. FALBE, "New Syntheses with Carbon Monoxide", Springer Verlag, Berlin, Heidelberg, New York, Seite 95 ff., (1980) beschrieben.

Die Reaktionstemperaturen für ein Hydroformylierungsverfahren mit den erfindungsgemäßen Bisphosphiten bzw. Bisphosphitmetallkomplexen als Katalysator liegen zwischen 40 °C und 180 °C, vorzugsweise zwischen 75 °C und 140 °C. Die Drücke, unter denen die Hydroformylierung abläuft, betragen 1-300 bar Synthesegas, vorzugsweise 15-64 bar. Das Molverhältnis zwischen Wasserstoff und Kohlenmonoxid (H₂/CO) im Synthesegas beträgt 10/1 bis 1/10, bevorzugt 1/1 bis 2/1.

Der Katalysator bzw. der Ligand ist homogen im Hydroformylierungsgemisch, bestehend aus Edukt (Olefine) und Produkten (Aldehyden, Alkoholen, im Prozess gebildete Hochsieder), gelöst. Optional kann zusätzlich ein Lösungsmittel verwendet werden.

Die Edukte für die Hydroformylierung sind Monoolefine oder Gemische von Monoolefinen mit 2 bis 25 Kohlenstoffatomen mit end- oder innenständiger C-C-Doppelbindung. Sie können geradkettig, verzweigt oder von cyclischer Struktur sein und auch mehrere olefinisch ungesättigte Gruppen aufweisen. Beispiele sind Propen, 1-Buten, c-2-Buten, t-2-Buten, Isobuten, Butadien, Mischungen der C4-Olefine, 1- oder 2-Penten, 2-Methylbuten-1, 2-Methylbuten-2, 3-Methylbuten-1, 1-, 2-oder 3-Hexen, das bei der Dimerisierung von Propen anfallende C6-Olefingemisch (Dipropen), 1-Hepten, Heptene, 2- oder 3-Methyl-1-hexen, 1-Octen, Octene, 2-Methylheptene, 3-Methylheptene, 5-Methylhepten-2, 6-Methylhepten-2, 2-Ethylhexen-1, das bei der Dimerisierung von Butenen anfallende isomere C8-Olefingemisch (Dibuten), 1-Nonen, Nonene, 2- oder 3-Methyloctene, das bei der Trimerisierung von Propen anfallende C9-Olefingemisch (Tripropen), Decene, 2-Ethyl-1-octen, Dodecene, das bei der Tetramerisierung von Propen oder der Trimerisierung von Butenen anfallende C12-Olefingemisch (Tetrapropen oder Tributen), Tetradecene, Hexadecene, bei der Tetramerisierung von Butenen anfallende C16-Olefingemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher C-Zahl. Ebenfalls können Olefine oder Olefingemische, die durch Fischer-Tropsch-Synthese erzeugt werden, eingesetzt werden, sowie Olefine, die durch Oligomerisierung von Ethen erhalten werden oder die über Methathesereaktionen oder Telomerisationsreaktion zugänglich sind.

Bevorzugte Edukte sind Propen, 1-Buten, 2-Buten, 1-Hexen, 1-Octen, Dimere und Trimere des Butens (Dibuten, Di-n-buten, Di-iso-buten, Tributen) und allgemein α-Olefine.

Die Hydroformylierung kann kontinuierlich oder diskontinuierlich durchgeführt werden. Beispiele für technische Ausführungen sind Rührkessel, Blasensäulen, Strahldüsenreaktoren, Rohrreaktoren, oder Schlaufenreaktoren, die zum Teil kaskadiert und/oder mit Einbauten versehen sein können.

Die Reaktion kann durchgehend oder in mehreren Stufen erfolgen. Die Trennung der entstandenen Aldehydverbindungen und des Katalysators kann durch eine herkömmliche Methode, wie Fraktionierung, durchgeführt werden. Technisch kann dies beispielsweise über eine Destillation, über einen Fallfilmverdampfer oder einen Dünnschichtverdampfer erfolgen. Die gilt besonders, wenn der Katalysator in einem hochsiedenden Lösungsmittel gelöst von den niedriger siedenden Produkten abgetrennt wird. Die abgetrennte Katalysatorlösung kann für weitere Hydroformylierungen verwendet werden. Bei Einsatz niederer Olefine (z. B. Propen, Buten, Penten) ist auch ein Austrag der Produkte aus dem Reaktor über die Gasphase möglich.

Die folgenden Beispiele sollen die Erfindung erläutern, nicht aber ihren Anwendungsbereich beschränken, der sich aus den Patentansprüchen ergibt.

### Beispiele

Alle Präparationen wurden mit Standard-Schlenk-Technik unter Schutzgas durchgeführt. Die Lösungsmittel wurden vor Gebrauch über geeigneten Trocknungsmitteln getrocknet.
Das in der Synthese eingesetzte 2-Chlor-1,3-dioxa-2-phospha-anthracen-4-on wurde laut einer Literaturvorschrift synthetisiert (BE 667036, Farbwerke Hoechst AG, 1966 ;*Chem. Abstr*. **65** (1966) 13741d). Das 3-Chlor-2,4-dioxa-3-phosphaphenanthren-1-on wurde auf analoge Weise erhalten. 2-Chlor-2,3-dioxa-2-phosphanaphthalin-4-on (van Boom's Reagenz) ist kommerziell erhältlich.

### Beispiel 1

### Synthese der Vorstufen C-1 und C-2

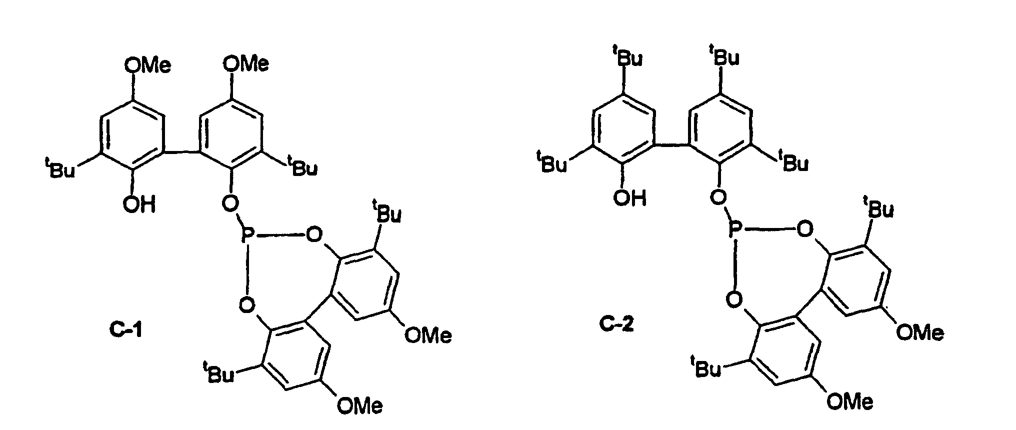

Vorstufe C-1

Zu einer Lösung von 2.42 g 2,2'-Bis(6-*tert*.-butyl-1-hydroxy-4-methoxyphenyl) (6.75 mmol) und 1.6 ml Pyridin in 22 ml THF tropft man bei 0 °C eine Lösung von 0.93 g PCl₃ (6.75 mmol) in 10 ml THF. Nach 4 h Rühren bei 25 °C wird das Lösungsmittel im Vakuum entfernt. Nach Zusatz von 40 ml Diethylether, Filtration und Einengen im Vakuum werden 2.8 g (98 %) an spektroskopisch reinem Chlorophosphorigsäureester des 2,2'-Bis(6-*tert*.-butyl-1-hydroxy-4-methoxyphenyl) erhalten: ³¹P-NMR (CD₂Cl₂) δ 172.7 ppm. 2.8 g dieses Chloroesters (6.62 mmol) in 20 ml THF gibt man bei Raumtemperatur zu einer bei -20 °C erhaltenen Monolithiumphenolatlösung aus 2.37 g 2,2'-Bis(6-*tert*.-butyl-1-hydroxy-4-methoxyphenyl) (6.62 mmol) in 30 ml THF und 20.7 ml einer 0.32 *M* Hexanlösung von n-Butyllithium (6.62 mmol). Nach 24 h wird im Vakuum eingeengt. Zugabe von 40 ml Methylenchlorid, Filtration und Entfernen des Solvens im Vakuum ergeben 4.6 g (93 %) an hochviskosem Produkt.
Analyse (ber. für C₄₄H₅₇O₈P = 744.9 g/ Mol) C 70.35 (70.95); H 7.86 (7.71). ³¹P-NMR (CD₂Cl₂) δ 140.7 ppm. ¹H-NMR (CD₂Cl₂) δ 1.43 (s, 9 H); 1.56 (s, 9 H); 1.63 (s, 9 H); 1.67 (s, 9 H); 4.01 (s, 3 H);4.03 (s, 6 H); 4.05 (s, 3 H); 5.42 (s, 1 H); 6.7...7.3 (m, 8 H) ppm. FAB MS: *m*/*e* 745 (37%, *M*⁺); 387 (100 %, *M*⁺-2,2'-Bis(6-*tert*.-butyl-1-hydroxy-4-methoxyphenyl)). IR (CHCl₃, 0.1 mm CaF₂), ν (OH) = 3549 cm⁻¹.

### Vorstufe C-2

Die Synthese wird analog zur Präparation von C-1 durchgeführt. Der Chlorophosphitdiester wird in nahezu quantitativer Ausbeute (98.4 %, ³¹P NMR, CD₂Cl₂ δ 172.0) erhalten. Für den zweiten Schritt der Reaktionssequenz werden dieser Chlorophosphitdiester (10.7 g, 22.5 mmol), 1.6 M Butyllithiumlösung in Hexan (14.1 ml) und die entsprechende Dihydroxydiphenylverbindung umgesetzt. Nach Entfernen des Lösungsmittels wird der Rückstand mehrmals mit heißem Hexan extrahiert. Aus den vereinigten Hexanfraktionen kristallisiert das Produkt aus, wird isoliert und im Vakuum getrocknet. Ausbeute: 76.4%
Analyse (ber. für C₅₆H₈₁O₄P = 849.23 g/ Mol) C 78.78 (79.20); H 9.95 (9.61). ³¹P-NMR (CD₂Cl₂) δ 142.3 ppm. ¹H-NMR (CD₂Cl₂) δ 0.98 (s, 9 H); 1.15 (s, 9 H); 1.21 (s, 9 H); 1.22 (s, 9 H); 1.23 (s, 9 H); 1.24 (s, 9 H); 1.30 (s, 9 H); 1.36 (s, 9 H); 5.35 (s, 1 H); 6.99 (d, 1 H); 7.01 (d, 1 H); 7.05 (d, 1 H); 7.06 (d, 1 H); 7.26 (d, 2 H); 7.32 (d, 1 H); 7.36 (d, 1 H) ppm.

### Beispiel 2

### Synthese von Ligand 2-a

Zu einer Lösung von 2.27 g **C-1** (3.04 mmol) in 24 ml THF werden bei -20°C unter Rühren innerhalb von 10 min 9.5 ml einer 0.32 *M* Lösung von n-Butyllithium (3.04 mmol) getropft. Nach Erwärmen auf Raumtemperatur wird zunächst 30 min nachgerührt, und die erhaltene Mischung dann zu 22 ml einer 0.138 *M* Lösung von 2-Chlor-1,3-dioxa-2-phospha-anthracen-4-on (3.04 mmol) in THF gegeben. Man rührt die Reaktionsmischung 4 h bei 25 °C, entfernt das Lösungsmittel im Vakuum und verrührt den sirupösen Rückstand 2 h mit 60 ml Hexan. Man filtriert, wäscht mit 2x 7 ml Hexan und extrahiert den Filterkuchen durch Rückdestillation von Hexan aus dem Filtrat. 3-tägiges Lagern der Mutterlauge bei 5 °C ergibt 0.828 g reinen Feststoff. Eine zusätzliche Extraktion des Filterkuchens der Hexanextraktion mit 35 ml siedendem Diethylether ergibt nach Volumenreduktion des Filtrates auf 50 % und Lagerung bei 5 °C 0.6 g Produkt. Gesamtausbeute: 1.428 g = 49 %. Analyse (ber. für C₅₅H₆₂O₁₁P₂ = 961.03 g/mol) C 68.69 (68.74); H 6.73 (6.50); P 6.41 (6.45) %. ³¹P-NMR (CD₂Cl₂): δ 118.1; 119.1; 139.0; 140.2. ¹H-NMR (CD₂Cl₂): δ 1.15..1.44 (36 H); 3.81..3.93 (12 H); 6.57..8.71(14 H).
FAB-MS: *m*/*e* 961 (30 %, *M*⁺); 745 (31 %); 727 (97 %); 387 (100 %).

### Beispiel 3

### Synthese von Ligand 3-a

Als P-Cl Verbindung kommt 3-Chlor-2,4-dioxa-3-phospha-phenanthren-1-on zum Einsatz. Die Synthese wird ausgehend von 2.31 g **C-1** ( 3.10 mmol) bis zur Extraktion des Filterkuchens mit rückdestilliertem Hexan aus dem Filtrat analog zur Darstellung von 2-a durchgeführt. Die anschließende Lagerung der Lösung bei 5 °C ergibt zunächst 0.90 g, nach Volumenreduktion auf die Hälfte weitere 1.36 g Produkt, Gesamtausbeute: 2.26 g = 75 %. Analyse (ber. für C₅₅H₆₂O₁₁P₂ = 961.03 g/mol) C 69.42 (68.74); H 7.16 (6.50); P 5.98 (6.45) %. ³¹P-NMR (CD₂Cl₂): δ 120.3; 121.1; 139.7; 140.7. ¹H-NMR (CD₂Cl₂): 0.87..1.40 (36 H); 3.75..3.88 (12 H); 6.63..8.17 (14 H). CI-MS: *m*/*e* 962 (31 %, *M-H*⁺); 745 (100 %); 405 (90 %); 387 (80 %).

### Beispiel 4

### Synthese von Ligand 6-a

Als P-Cl Verbindung kommt 2-Chlor-1.3-dioxa-2-phosphanaphthalin-4-on zum Einsatz. Die Synthese wird ausgehend von 6.86 g **C-1** bis zur Extraktion des Filterkuchens analog zur Darstellung von 2-a durchgeführt. Die Extraktion erfolgt mit heißem Hexan und mit Diethylether. Nach Reduzierung der Lösungsmittelmenge auf ein Drittel und anschließender Lagerung der Lösung bei -20 °C erhält man das Produkt in 54% Ausbeute. ³¹P-NMR (CD₂Cl₂): δ 119.2 (m); 119.8 (m); 139.5 (m); 140.1 (m); ¹H-NMR (CD₂Cl₂): 1.02..1.26 (36 H); 3.67..3.74 (12 H); 6.43..7.99 (12 H). FAB-MS: m/e 911 (100%, M+), 744 (18%), 387 (13%).

### Beispiel 5

### Synthese von Ligand 6-b

Als P-Cl Verbindung kommt 2-Chlor-1.3-dioxa-2-phosphanaphthalin-4-on zum Einsatz. Die Synthese wird ausgehend von 4.93 g **C-2** analog zur Synthese von Verbindung 2-a durchgeführt. Gesamtausbeute 50.4 %. Analyse (ber. für C63H₈₄O₇P₂ = 1015.30 g/mol) C 74.86 (74.53); H 8.43 (8.34). ³¹P-NMR (CD₂Cl₂): δ 118.5, 119.7, 142.0, 142.8; ¹H-NMR (CD₂Cl₂): 0.90..1.36 (72 H); 6.74..7.90 (12 H); FAB-MS: m/e 1015 (7%, M+), 832 (100%), 439 (70%).

### Beispiel 6

### Synthese von Ligand 2-b

Als P-CI Verbindung kommt 2-Chlor-1,3-dioxa-2-phospha-anthracen-4-on zum Einsatz. Die Synthese wird ausgehend von 5.07 g C-2 analog zur Darstellung von 2-a durchgeführt. Ausbeute: 73 %. Analyse (ber. für C₆₇H₈₆O₇P₂ = 1065.36 g/mol) C 75.24 (75.54); H 8.16 (8.14). ³¹P-NMR (CD₂Cl₂): δ 117.8, 118.9, 142.1, 142.9; Verhältnis der Diastereomeren 1.3 : 1. ¹H-NMR (CD₂Cl₂): 0.99..1.35 (72 H); 6.95..8.55 (14 H). FAB-MS: m/e 1064 (18%, M-H), 831 (100%), 439 (78%).

### Beispiel 7 und 8

### Hydroformylierung von 1-Octen

Die Versuchsdurchführung erfolgte nach Befüllen unter Schutzgas in einem mit. Begasungsrührer, Druckpipette und Nachdruckregler ausgestatteten 200 ml-Edelstahlautoklav der Fa. Buddeberg, Mannheim, im Ölbadthermostaten. Zur Minimierung eines Einflusses von Feuchtigkeit und Sauerstoff wurde das als Solvens benutzte Toluol mit Natrium-Ketyl getrocknet und unter Argon destilliert. Das als Substrat eingesetzte 1-Octen wurde mehrere Stunden über Natrium am Rückfluß erhitzt und unter Argon destilliert.
Der Autoklav wurde beschickt mit 27 ml Toluol, in welchem 5.456 mg= 0.0176 mmol [acacRh(COD)], und 0.088 mmol des jeweiligen Liganden gelöst waren. Das molare Verhältnis Rh/P betrug damit 1:10. In die Druckpipette über dem Reaktor gab man 24 ml = ca. 16.8 g (149.3 mmol) 1-Octen. Das Verhältnis Rh/1-Octen betrug damit ca. 1: 8500. Reaktor und Druckpipette wurden über einen der Druckregelstrecke parallel geschalteten Bypass bei einem Solldruck von 50 bar mit 33 bar, bei einem Solldruck von 20 bar mit 13 bar CO/H₂ (1:1; Synthesegas) beaufschlagt und der Reaktorinhalt unter magnetischem Rühren mit dem Begasungsrührer mit 1500 min⁻¹ auf 80 bzw. 100°C temperiert. Nach Erreichen der Solltemperatur wurde der Druck auf 47 bar (17 bar) erhöht und das Olefingemisch aus der Druckpipette mit einem Druck von 55 bar (25 bar) in den Reaktor gepreßt. Es stellte sich ein Anfangsdruck der Reaktion von 49.6 bar (19.2 bar) ein. Nach sofortiger manueller Regulierung auf 50 bar (20 bar) wurde der Bypass geschlossen, und der Druck über die gesamte Reaktionszeit mit dem Nachdruckregler konstant gehalten. Der Versuch wurde unter Zwangskühlung nach Ablauf der festgelegten Reaktionszeit beendet. Die Reaktionslösung wurde unter Schutzgas entnommen und gaschromatografisch analysiert.

Die nachfolgende Tabelle enthält die mit den einzelnen Liganden erhaltenen Ergebnisse.

| **Beispiel** | **Ligand** | **Temp.** **[°C]** | **p** **[bar]** | **t** **[h]** | **Ausbeute** **[%]** | **Nonanal Anteil [%]** |
|---|---|---|---|---|---|---|
| **7** | **2-a** | 100 | 20 | 3 | 81 | 79.0 |
| **8** | **3-a** | 100 | 20 | 3 | 79 | 83.8 |

### Beispiel 9 bis 19

### Hydroformylierung einer Mischung von 1-Octen, 2-Octen, 3-Octen und 4-Octen

Die Versuchsdurchführung erfolgte nach Befüllen unter Schutzgas in einem mit Begasungsrührer, Druckpipette und Nachdruckregler ausgestatteten 200 ml-Edelstahlautoklav der Fa. Buddeberg, Mannheim, im Ölbadthermostaten. Zur Minimierung eines Einflusses von Feuchtigkeit und Sauerstoff wurde das als Solvens benutzte Toluol mit Natrium-Ketyl getrocknet und unter Argon destilliert. Das als Substrat eingesetzte Octenisomerengemisch wurde mehrere Stunden über Natrium am Rückfluß erhitzt und unter Argon destilliert. Zusammensetzung: 1-Octen, 3.3 %; *cis*+*trans*-2-Octen, 48.5 %; *cis*+*trans*-3-Octen, 29.2%; *cis*+*trans*-Octen-4, 16.4 %; verzweigte C8-Olefine, 2.6 %.
Der Autoklav wurde beschickt mit 41 ml Toluol, in welchem 18.75 mg= 0.0604 mmol [acacRh(COD)], der jeweilige Bidentatligand und ggf. der nachfolgend abgebildete Coligand gelöst waren. Das Verhältnis Rh/Bidentatligand (Ligand)/Etherphosphonit (Coligand) ist in der Tabelle angegeben. In die Druckpipette über dem Reaktor gab man 15 ml = 10.62 g (94.63 mmol) Octene. Das Verhältnis Rh/Octene betrug damit ca. 1: 1570. Reaktor und Druckpipette wurden über einen der Druckregelstrecke parallel geschalteten Bypass mit 13 bar CO/H₂ (1:1; Synthesegas) beaufschlagt und der Reaktorinhalt unter magnetischem Rühren mit dem Begasungsrührer mit 1500 min⁻¹ auf 130 °C temperiert. Nach Erreichen der Solltemperatur wurde der Druck auf 17 bar erhöht, und das Olefingemisch aus der Druckpipette mit einem Druck von 25 bar in den Reaktor gepreßt. Es stellte sich ein Anfangsdruck der Reaktion von 19.2 bar ein. Nach sofortiger manueller Regulierung auf 20 bar wurde der Bypass geschlossen, und der Druck über die gesamte Reaktionszeit mit dem Nachdruckregler konstant gehalten. Der Versuch wurde unter Zwangskühlung nach drei Stunden beendet. Die Reaktionslösung wurde unter Schutzgas entnommen und gaschromatografisch analysiert.

Als Coligand wurde eingesetzt:

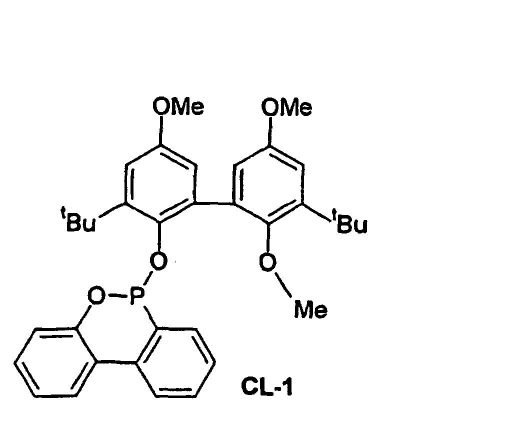

Die nachfolgende Tabelle enthält die mit den einzelnen Liganden erhaltenen Ergebnisse.

| **Beispiel** | **Ligand / Coligand** | **T** **[°C]** | **Rh/Lig/CoLig/Olefin [mol/mol/mol/mol]** | **t** **[h]** | **Ausb.** **[%]** | **Nonanal Anteil [%]** |
|---|---|---|---|---|---|---|
| **9** | **2-a** | 130 | 1/5/0/1570 | 3 | 95 | 64.2 |
| **10** | **2-b** | 130 | 1/5/0/1570 | 3 | 93 | 67.9 |
| **11** | **3-a** | 130 | 1/5/0/1570 | 3 | 96 | 69.0 |
| **12** | **6-a** | 130 | 1/5/0/1570 | 3 | 94 | 63.9 |
| **13** | **6-b** | 130 | 1/5/0/1570 | 3 | 95 | 67.3 |
| **14** | **2-a/CL-1** | 130 | 1/2.5/5/1570 | 3 | 92 | 63.5 |
| **15** | **3-a/CL-1** | 130 | 1/2.5/5/1570 | 3 | 93 | 67.8 |
| **16** | **6-a/CL-1** | 130 | 1/2.5/5/1570 | 6 | 98 | 63.0 |
| **17**^{**#**} | **3-a** | 130 | 1/5/0/15700 | 6 | 74 | 69.5 |
| **18**^{**#**} | **6-a** | 130 | 1/510/15700 | 6 | 83 | 64.1 |
| **19**^{**#**} | **6-b** | 130 | 1/5/0/15700 | 6 | 66 | 69.0 |

| | | | | | | |
|---|---|---|---|---|---|---|
| ^{#} 0.00604 mmol [acacRh(COD)], entsprechend weniger Liganden | | | | | | |

### Beispiel 20-25

### Hydroformylierung von technischem Di-n-Buten

Die Versuchdurchführung erfolgte analog zu den Beispielen 9-19 mit 15 ml = 10.70 g (95.34 mmol) einer Mischung von doppelbindungs- und gerüstisomeren Octenen, die durch Dimerisierung von n-Butenen erhalten wurden. Die nachfolgende Tabelle enthält sowohl Ergebnisse, die mit reinen Bidentatliganden, als auch unter Anwendung einer Mischung von Bidentatligand/Coligand CL-1 erhalten wurden.

| **Beispiel** | **Ligand / Coligand** | **T** **[°C]** | **Rh/Lig/CoLig/Olefin [mol/mol/mol/mol]** | **t** **[h]** | **Ausb.** **[%]** | **Nonanal Anteil [%]** |
|---|---|---|---|---|---|---|
| **20** | **2-a** | 130 | 1/5/0/1578 | 6 | 59 | 63.2 |
| **21** | **2-a / CL-1** | 130 | 1/2.5/5/1578 | 6 | 57 | 63.0 |
| **22** | **3-a** | 130 | 1/5/0/1578 | 6 | 56 | 65. 7 |
| **23** | **3-a / CL-1** | 130 | 1/2.5/5/1578 | 6 | 60 | 65.4 |
| **24** | **6-a** | 130 | 1/5/0/1578 | 6 | 56 | 63.1 |
| **25** | **6-a / CL-1** | 130 | 1/2.5/5/1578 | 6 | 58 | 62.3 |

## Patentansprüche

1. Bisphosphit der Formel I 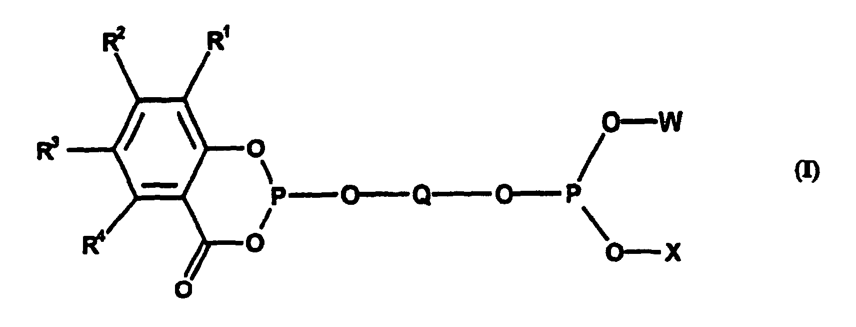 mit R¹, R², R³, R⁴ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, wobei R¹ bis R⁴ eine gleiche oder unterschiedliche Bedeutung besitzen und kovalent miteinander verknüpft sein können,
R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen, die gleich oder unterschiedlich oder kovalent miteinander verknüpft sein können.

2. Bisphospite der Formel II, III und IV 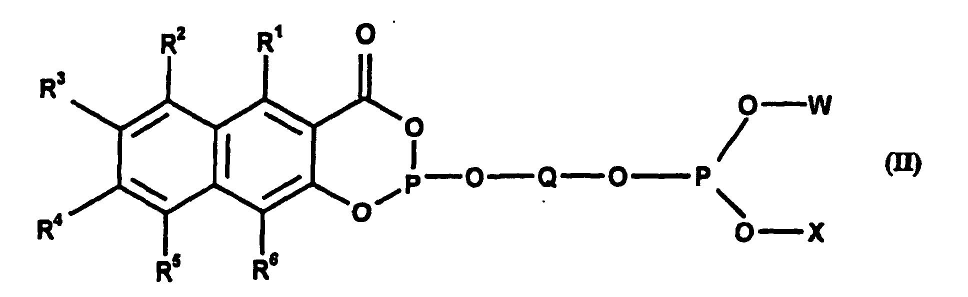 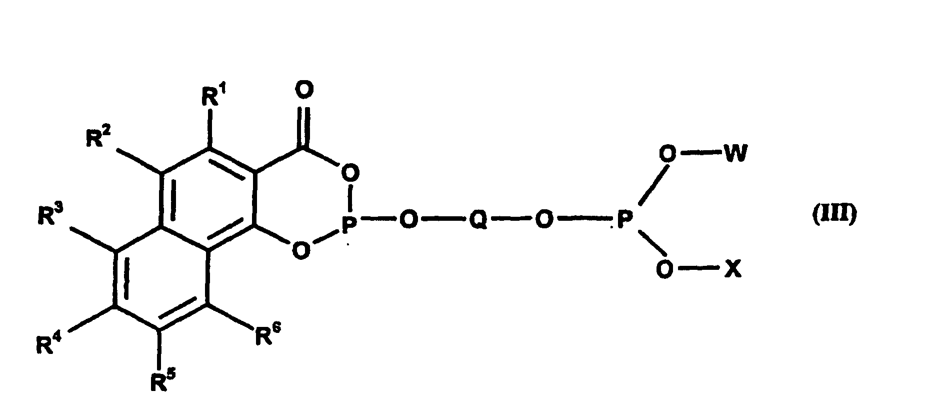 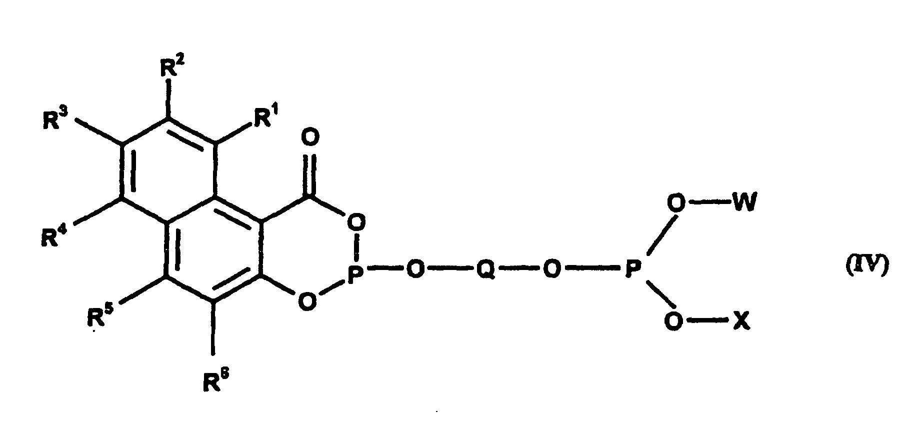 mit R¹, R², R³, R⁴, R⁵, R⁶ = H aliphatischer, alicyclischer, aliphatisch-alicyclischer, heterocyclischer, aliphatisch-heterocyclischer, aromatisch-aromatischer, aromatischer, aliphatisch-aromatischer Kohlenwasserstoffrest mit 1 bis 50 Kohlenstoffatomen, F, Cl, Br, I, -CF₃, -OR⁷, -COR⁷, -CO₂R⁷, -CO₂M,-SR⁷, -SO₂R⁷, -SOR⁷, -SO₃R⁷, -SO₃M, -SO₂NR⁷R⁸, NR⁷R⁸, N=CR⁷R⁸, NH₂, wobei R¹ bis R⁶ eine gleiche oder unterschiedliche Bedeutung besitzen, R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen, die gleich oder unterschiedlich oder kovalent miteinander verknüpft sein können.

3. Bisphosphit gemäß Anspruch 1 oder 2,
**dadurch gekennzeichnet,**
**dass** W und X aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen, mit einer kovalenten Verknüpfung gemäß Formel V 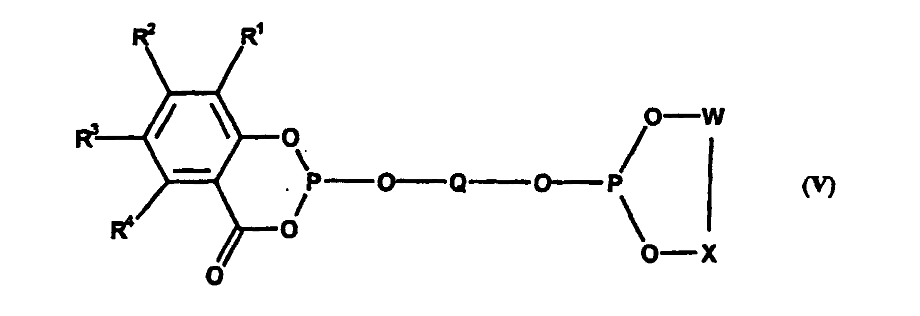 sind und R¹, R², R³, R⁴ und Q die in Anspruch 1 genannten Bedeutungen und Maßgaben besitzen.

4. Bisphosphit gemäß Anspruch 1 oder 2,
**dadurch gekennzeichnet,**
**dass** W und X aromatische Kohlenwasserstoffreste mit 1 bis 50 Kohlenstoffatomen mit kovalenten Verknüpfungen gemäß Formel VI 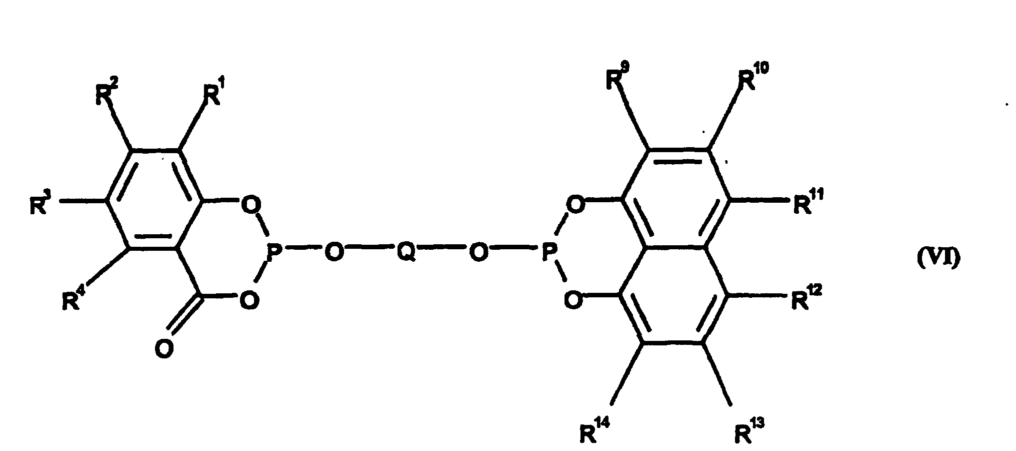 sind,
mit R⁹, R¹⁰, R¹¹, R¹², R¹³, R¹⁴ = H, aliphatischer, alicyclischer, aliphatisch-alicyclischer, heterocyclischer, aliphatisch-heterocyclischer, aromatisch-aromatischer, aromatischer, aliphatisch-aromatischer Kohlenwasserstoffrest mit 1 bis 50 Kohlenstoffatomen, F, Cl, Br, I, -CF₃, -OR²⁵, -COR²⁵, -CO₂R²⁵, -CO₂M,-SR²⁵,
-SO₂R²⁵, -SOR²⁵, -SO₃R²⁵, -SO₃M, -SO₂NR²⁵R²⁶, NR²⁵R²⁶, N=CR²⁵R²⁶, NH₂, wobei R⁹ bis R¹⁴ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴ und Q die in Anspruch 1 genannten Bedeutungen und Maßgaben besitzen.

5. Bisphosphit gemäß Anspruch 1 oder 2,
**dadurch gekennzeichnet,**
**dass** W und X aromatische Kohlenwasserstoffreste mit 1 bis 50 Kohlenstoffatomen mit einer kovalenten Verknüpfung gemäß Formel VII 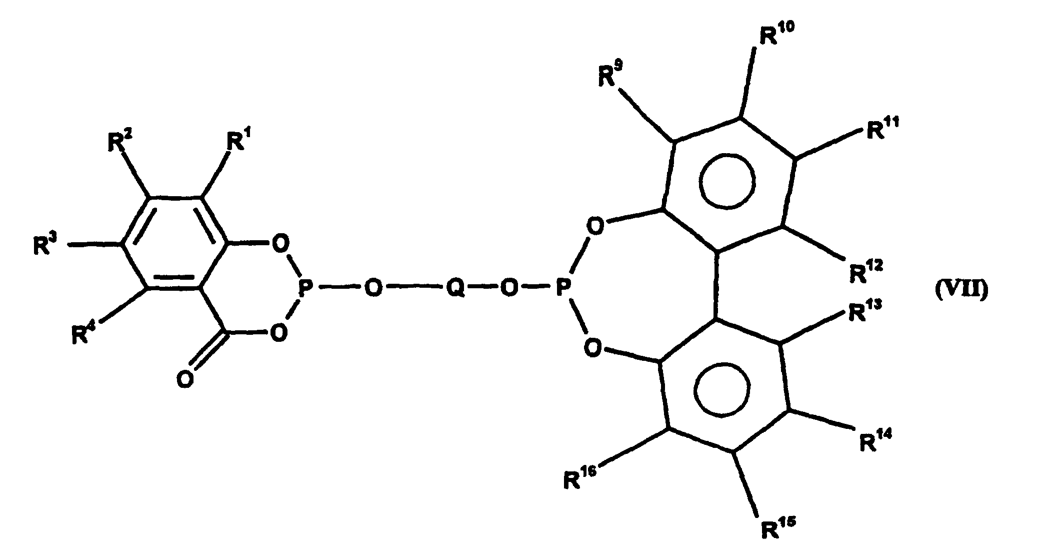 sind,
mit R⁹, R¹⁰, R¹¹, R¹², R¹³, R¹⁴, R¹⁵, R¹⁶ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR²⁵, - COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R⁹ bis R¹⁶ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴ und Q die in Anspruch 1 genannten Bedeutungen und Maßgaben besitzen.

6. Bisphosphit nach einem der Ansprüche 1 bis 5,
**dadurch gekennzeichnet,**
**dass** das Q ein Kohlenwasserstoffrest gemäß Formel VIII 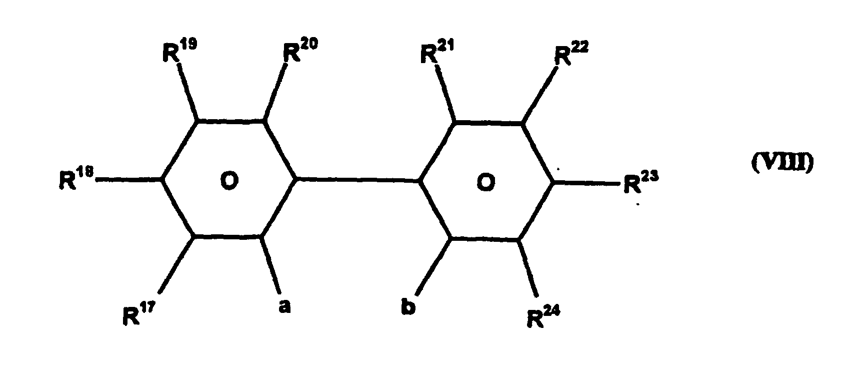 ist,
mit R¹⁷, R¹⁸, R¹⁹, R²⁰, R²¹, R²², R²³, R²⁴ = H, aliphatischer, alicyclischer, aliphatisch-alicyclischer, heterocyclischer, aliphatisch-heterocyclischer, aromatisch-aromatischer, aromatischer, aliphatisch-aromatischer Kohlenwasserstoffrest mit 1 bis 50 Kohlenstoffatomen, F, Cl, Br, I, -CF₃, -OR²⁵, -COR²⁵, - CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R¹⁷ bis R²⁴ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion
wobei die Positionen a und b als Anknüpfpunkte dienen.

7. Bisphosphitmetallkomplex, enthaltend ein Metall der 4., 5., 6., 7. oder 8. Nebengruppe des Periodensystems der Elemente und ein oder mehrere Bisphosphite der Formel I 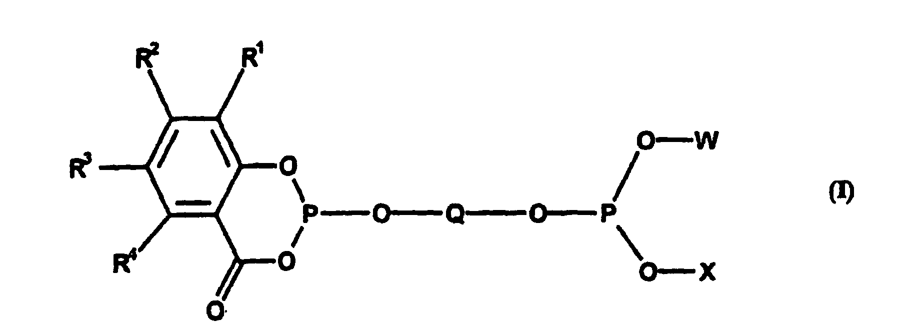 mit R¹, R², R³, R⁴ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, wobei R¹ bis R⁴ eine gleiche oder unterschiedliche Bedeutung besitzen und kovalent miteinander verknüpft sein können.
R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, die gleich oder unterschiedlich oder kovalent miteinander verknüpft sein können.

8. Bisphosphitmetallkomplex, enthaltend ein Metall der 4., 5., 6., 7. oder 8. Nebengruppe des Periodensystems der Elemente und ein oder mehrere Bisphosphite der Formeln II, III und/oder IV 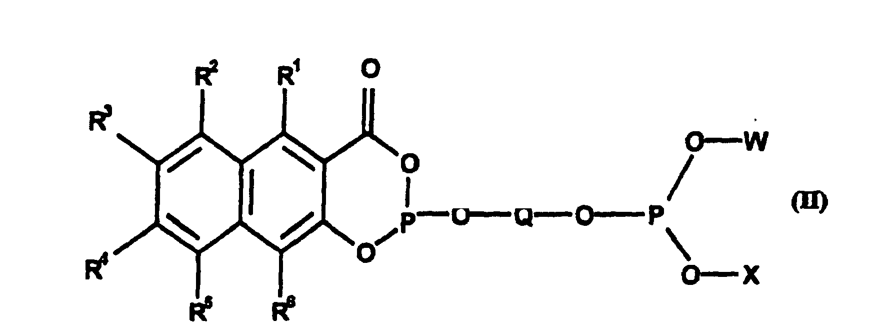 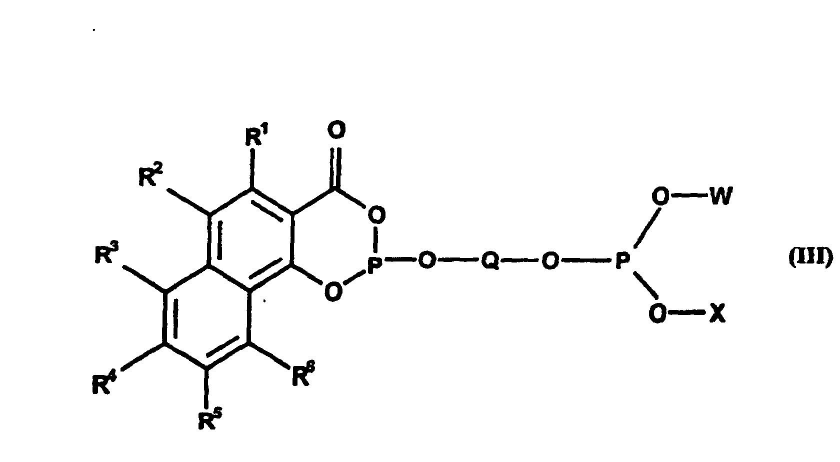 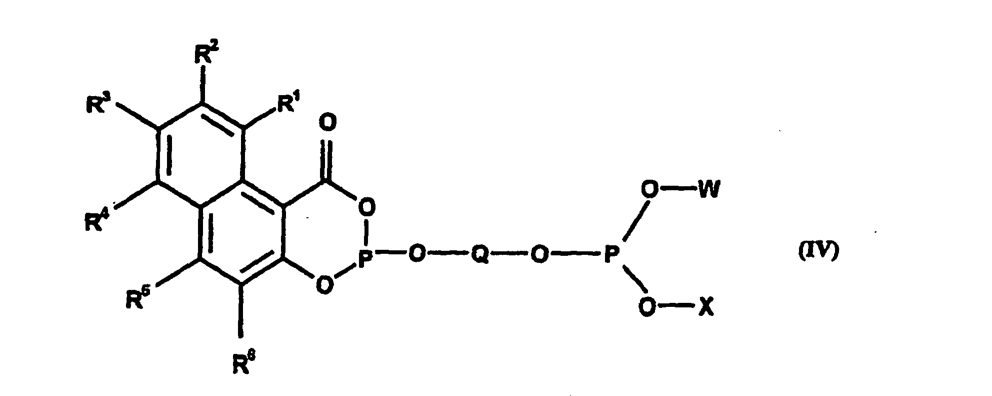 mit R¹, R², R³, R⁴, R⁵, R⁶ = H aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, wobei R¹ bis R⁶ eine gleiche oder unterschiedliche Bedeutung besitzen,
R⁷, R⁸ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, die gleich oder unterschiedlich oder kovalent miteinander verknüpft sein können.

9. Bisphosphitmetallkomplex nach Anspruch 7 oder 8,
**dadurch gekennzeichnet,**
**dass** W und X aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen, mit einer kovalenten Verknüpfung gemäß Formel V 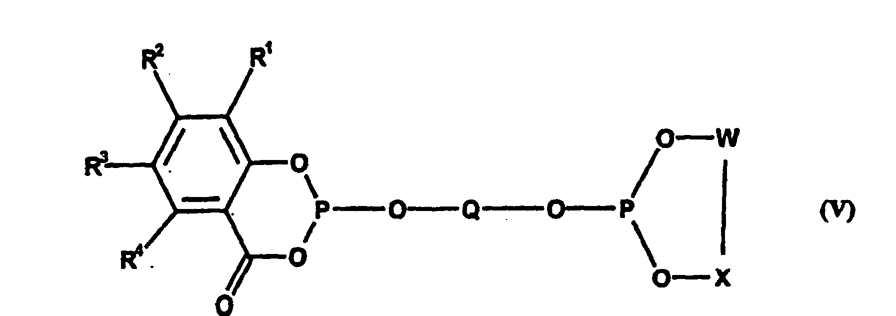 sind und R¹, R², R³, R⁴ und Q die in Anspruch 7 genannten Bedeutungen und Maßgaben besitzen.

10. Bisphosphitmetallkomplex nach Anspruch 7 oder 8,
**dadurch gekennzeichnet,**
**dass** W und X aromatische Kohlenwasserstoffreste mit 1 bis 50 Kohlenstoffatomen mit kovalenten Verknüpfungen gemäß Formel VI 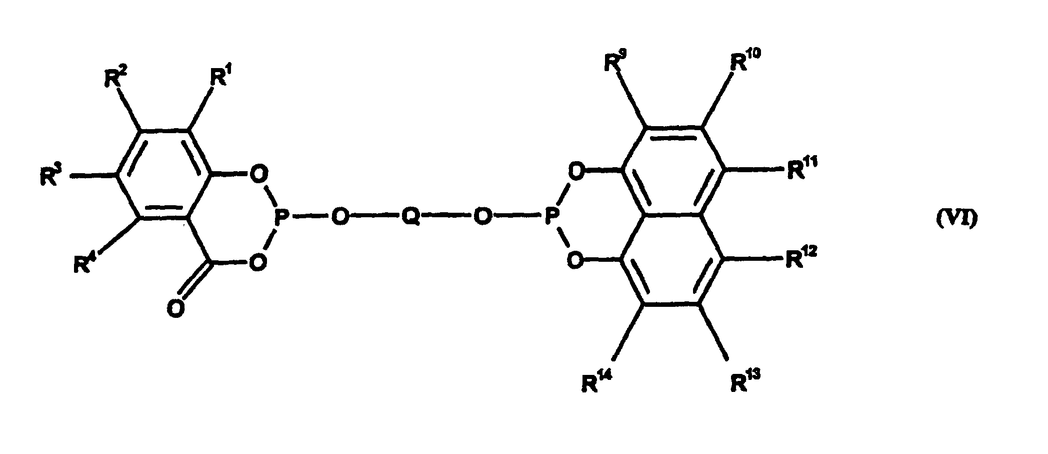 sind,
mit R⁹, R¹⁰, R¹¹, R¹², R¹³, R¹⁴ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R⁹ bis R¹⁴ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴ und Q die in Anspruch 1 genannten Bedeutungen und Maßgaben besitzen.

11. Bisphosphitmetallkomplex nach einem der Ansprüche 7 oder 8,
**dadurch gekennzeichnet,**
**dass** W und X aromatische Kohlenwasserstoffreste mit 1 bis 50 Kohlenstoffatomen mit einer kovalenten Verknüpfung gemäß Formel VII 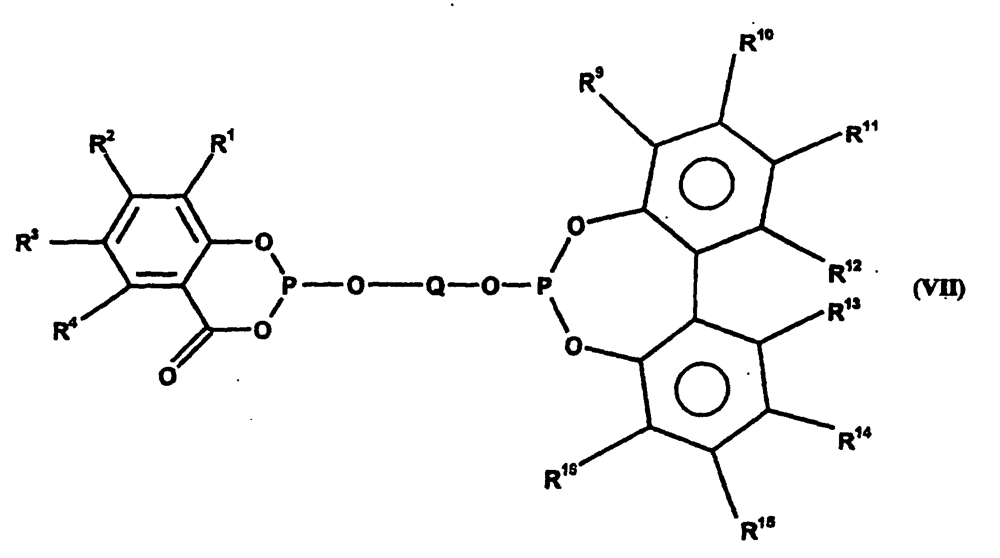 sind,
mit R⁹, R¹⁰, R¹¹, R¹², R¹³, R¹⁴, R¹⁵, R¹⁶ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR²⁵, - COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R⁹ bis R¹⁶ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴ und Q die in Anspruch 1 genannten Bedeutungen und Maßgaben besitzen.

12. Bisphosphitmetallkomplex nach einem der Ansprüche 7 bis 11,
**dadurch gekennzeichnet,**
**dass** das Q ein Kohlenwasserstoffrest gemäß Formel VIII 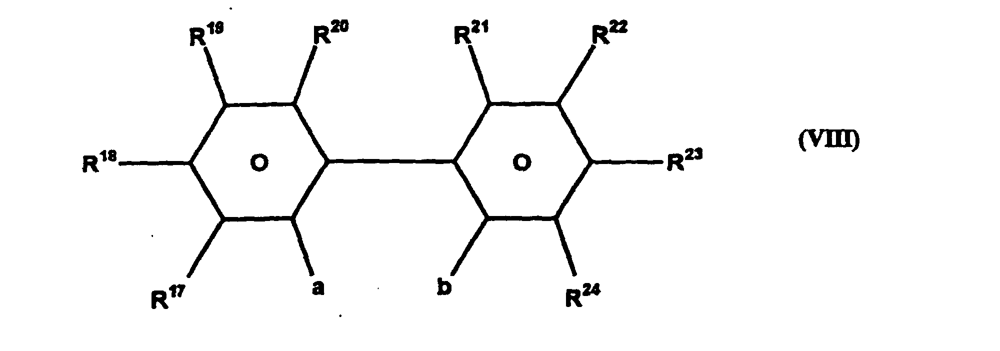 ist,
mit R¹⁷, R¹⁸, R¹⁹, R²⁰, R²¹, R²², R²³, R²⁴ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR²⁵, - COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, wobei R¹⁷ bis R²⁴ eine gleiche oder unterschiedliche Bedeutung besitzen und kovalent miteinander verknüpft sein können,
R²⁵, R²⁶ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion
wobei die Positionen a und b als Anknüpfpunkte dienen.

13. Bisphosphitmetallkomplex nach einem der Ansprüche 7 bis 12,
**dadurch gekennzeichnet,**
**dass** als Metall Rhodium, Platin, Kobalt oder Ruthenium eingesetzt wird.

14. Verwendung der Bisphosphite gemäß einem der Ansprüche 1 bis 6 in einem Verfahren zur Hydroformylierung von Olefinen.

15. Verwendung der Bisphosphitmetallkomplexe gemäß einem der Ansprüche 7 bis 12 in einem Verfahren zur Hydroformylierung von Olefinen.

16. Verwendung der Bisphosphite gemäß einem der Ansprüche 1 bis 6 in einem Verfahren zur Hydroformylierung von Olefinen unter Anwesenheit von weiteren phosphorhaltigen Liganden.

17. Verwendung der Bisphosphitmetallkomplexe gemäß einem der Ansprüche 7 bis 12 in einem Verfahren zur Hydroformylierung von Olefinen unter Anwesenheit von weiteren phosphorhaltigen Liganden.

## Claims

1. A bisphosphite of the formula I 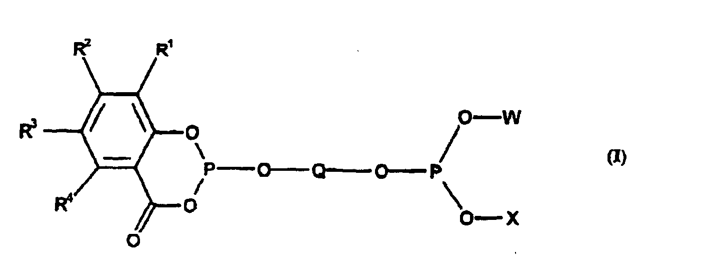 where R¹, R², R³, R⁴ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, F, Cl, Br, I, -CF₃, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, where R¹ to R⁴ are identical or different and may be covalently linked to one another,
R⁷, R⁸ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion,
Q = a divalent aliphatic, alicyclic, aliphatic-alicyclic, heterocyclic, aliphatic-heterocyclic, aromatic, aliphatic-aromatic hydrocarbon radical having from 1 to 50 carbon atoms,
W, X = aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms which may be identical or different or covalently linked to one another.

2. A bisphosphite of the formula II, III or IV 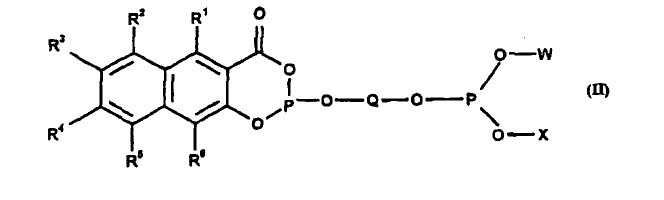 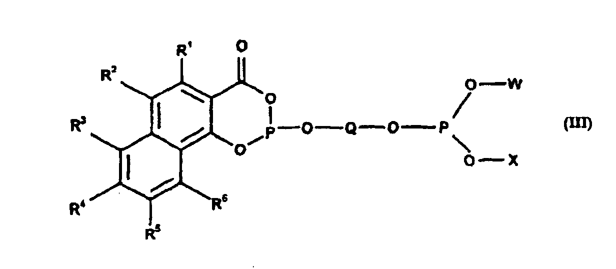 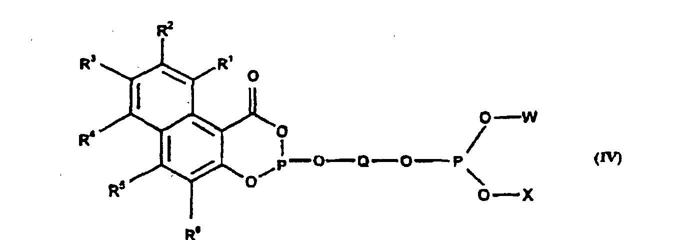 where R¹, R², R³, R⁴, R⁵, R⁶ = H, an aliphatic, alicyclic, aliphatic-alicyclic, heterocyclic, aliphatic-heterocyclic, aromatic-aromatic, aromatic, aliphatic-aromatic hydrocarbon radical having from 1 to 50 carbon atoms, F, Cl, Br, I, -CF₃, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, where R¹ to R⁶ are identical or different,
R⁷, R⁸ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion,
Q = a divalent aliphatic, alicyclic, aliphatic-alicyclic, heterocyclic, aliphatic-heterocyclic, aromatic, aliphatic-aromatic hydrocarbon radical having from 1 to 50 carbon atoms,
W, X = aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms which may be identical or different or covalently linked to one another.

3. A bisphosphite according to claim 1 or 2,
**characterized in that** W and X are aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms which are covalently linked as in formula V 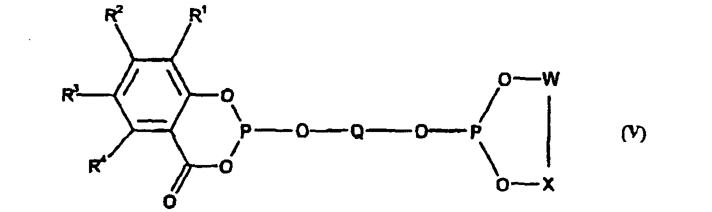 and R¹, R², R³, R⁴ and Q have the meanings and are subject to the provisos specified in claim 1.

4. A bisphosphite according to claim 1 or 2, **characterized in that** W and X are aromatic hydrocarbon radicals having from 1 to 50 carbon atoms which are covalently linked as in formula VI 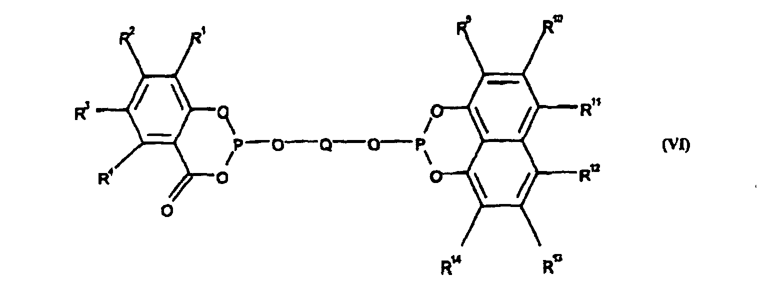 where R⁹, R¹⁰, R¹¹, R¹², R¹³, R¹⁴ = H, an aliphatic, alicyclic, aliphatic-alicyclic, heterocyclic, aliphatic-heterocyclic, aromatic-aromatic, aromatic, aliphatic-aromatic hydrocarbon radical having from 1 to 50 carbon atoms, F, Cl, Br, I, -CF₃, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, where R⁹ to R¹⁴ are identical or different and may be covalently linked to one another,
R²⁵, R²⁶ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion and
R¹, R², R³, R⁴ and Q have the meanings and are subject to the provisos specified in claim 1.

5. A bisphosphite according to claim 1 or 2, **characterized in that** W and X are aromatic hydrocarbon radicals having from 1 to 50 carbon atoms which are covalently linked as in formula VII 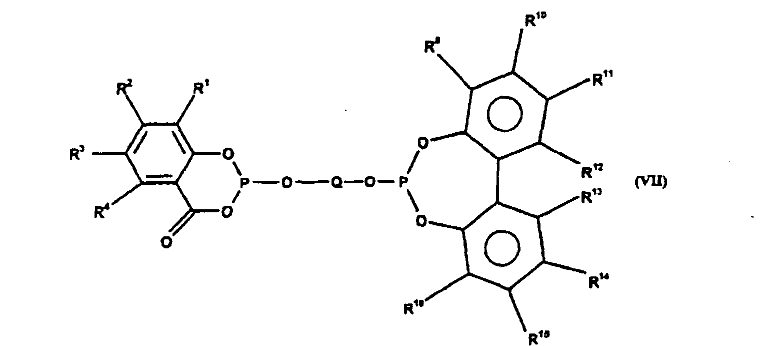 where R⁹, R¹⁰, R¹¹, R¹², R¹³, R¹⁴, R¹⁵, R¹⁶ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, F, Cl, Br, I, -CF₃, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, where R⁹ to R¹⁶ are identical or different and may be covalently linked to one another,
R²⁵, R²⁶ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion and
R¹, R², R³, R⁴ and Q have the meanings and are subject to the provisos specified in claim 1.

6. A bisphosphite according to any one of claims 1 to 5, **characterized in that** Q is a hydrocarbon radical of the formula VIII 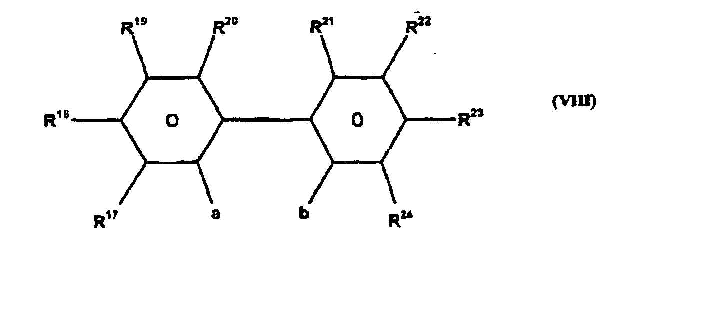 where R¹⁷, R¹⁸, R¹⁹, R²⁰, R²¹, R²², R²³, R²⁴ = H, an aliphatic, alicyclic, aliphatic-alicyclic, heterocyclic, aliphatic-heterocyclic, aromatic-aromatic, aromatic, aliphatic-aromatic hydrocarbon radical having from 1 to 50 carbon atoms, F, Cl, Br, I, -CF₃, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, where R¹⁷ to R²⁴ are identical or different and may be covalently linked to one another,
R²⁵, R²⁶ = H, a substituted or unsubstituted, aliphatic or aromatic hydrocarbon radical having from 1 to 25 carbon atoms,
M = an alkali metal, alkaline earth metal, ammonium or phosphonium ion,
where the positions a and b are linkage points.

7. A bisphosphite-metal complex comprising a metal of transition group 4, 5, 6, 7 or 8 of the Periodic Table of the Elements and one or more bisphosphites of the formula I 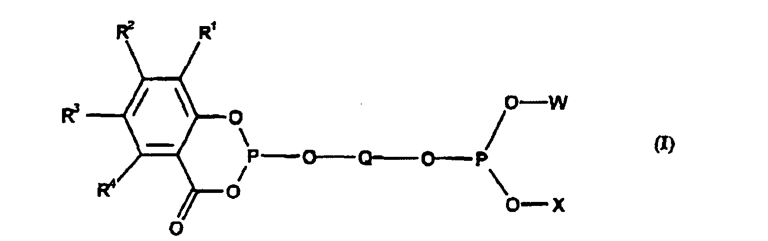 where R¹, R², R³, R⁴ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, F, Cl, Br, I, -CF₃, -OR⁷, - COR⁷, -CO₂R⁷, -CO₂M, -SR⁷, -SO₂R⁷, -SOR⁷, -SO₃R⁷, - SO₃M, -SO₂NR⁷R⁸, NR⁷R⁸, N=CR⁷R⁸, NH₂, where R¹ to R⁴ are identical or different and may be covalently linked to one another, R⁷, R⁸ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion,
Q = a divalent aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms,
W, X = aliphatic, alicyclic, aliphatic-alicyclic, heterocyclic, aliphatic-heterocyclic, aromatic, aliphatic-aromatic hydrocarbon radicals having from 1 to 50 carbon atoms which may be identical or different or covalently linked to one another.

8. A bisphosphite-metal complex comprising a metal of transition group 4, 5, 6, 7 or 8 of the Periodic Table of the Elements and one or more bisphosphites of the formulae II, III and/or IV 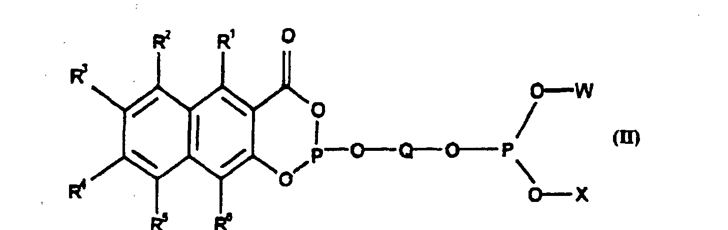 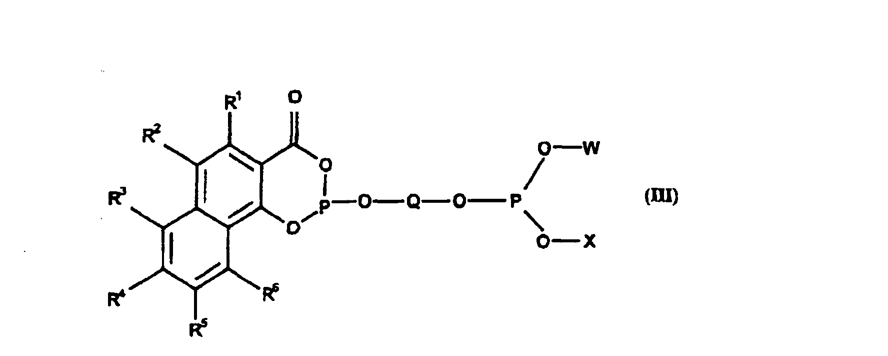 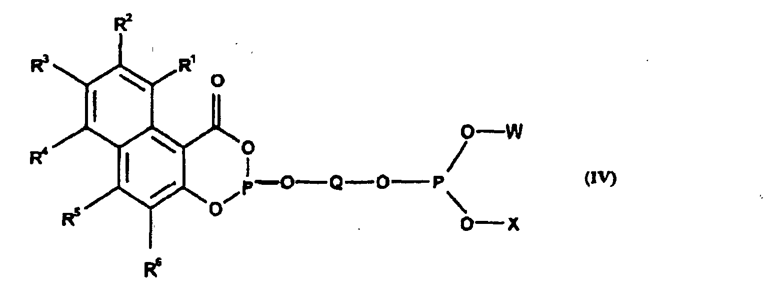 where R¹, R², R³, R⁴, R⁵, R⁶ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, F, Cl, Br, I, -CF₃, -OR⁷, -COR⁷, -CO₂R⁷, -CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, where R¹ to R⁶ are identical or different,
R⁷, R⁸ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion,
Q = a divalent aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms,
W, X = aliphatic, alicyclic, aliphatic-alicyclic, heterocyclic, aliphatic-heterocyclic, aromatic, aliphatic-aromatic hydrocarbon radicals having from 1 to 50 carbon atoms which may be identical or different or covalently linked to one another.

9. A bisphosphite-metal complex according to claim 7 or 8, **characterized in that** W and X are aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms which are covalentlv linked as in formula V 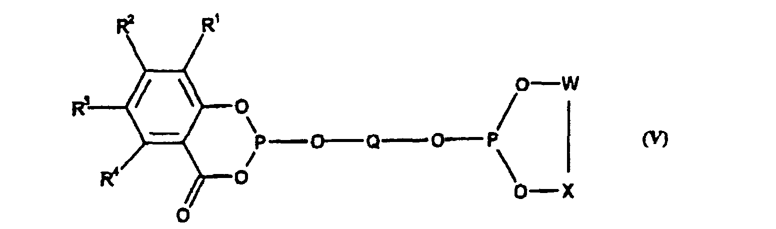 and R¹, R², R³, R⁴ and Q have the meanings and are subject to the provisos specified in claim 7.

10. A bisphosphite-metal complex according to claim 7 or 8, **characterized in that** W and X are aromatic hydrocarbon radicals having from 1 to 50 carbon atoms which are covalently linked as in formula VI 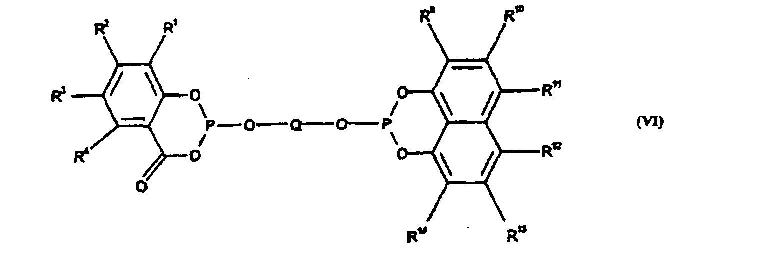 where R⁹, R¹⁰, R¹¹, R¹², R¹³, R¹⁴ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, F, Cl, Br, I, -CF₃, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, where R⁹ to R¹⁴ are identical or different and may be covalently linked to one another,
R²⁵, R²⁶ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion and
R¹, R², R³, R⁴ and Q have the meanings and are subject to the provisos specified in claim 1.

11. A bisphosphite-metal complex according to claim 7 or 8, **characterized in that** W and X are aromatic hydrocarbon radicals having from 1 to 50 carbon atoms which are covalently linked as in formula VII 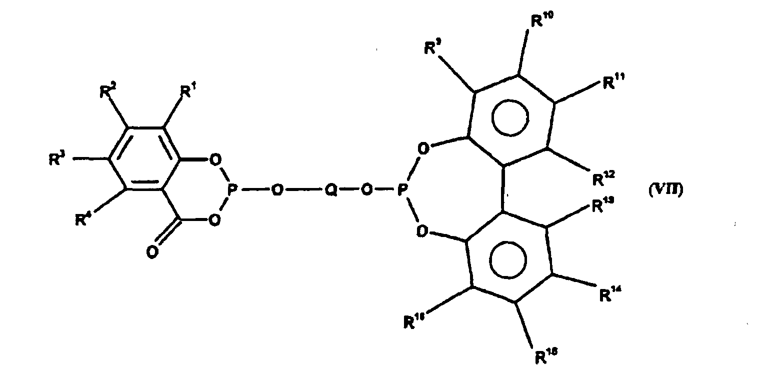 where R⁹, R¹⁰, R¹¹, R¹², R¹³, R¹⁴, R¹⁵, R¹⁶ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, F, Cl, Br, I, -CF₃, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, where R⁹ to R¹⁶ are identical or different and may be covalently linked to one another,
R²⁵, R²⁶ = H, a substituted or unsubstituted, aliphatic or aromatic hydrocarbon radical having from 1 to 25 carbon atoms, and may be identical or different,
M = an alkali metal, alkaline earth metal, ammonium or phosphonium ion and
R¹, R², R³, R⁴ and Q have the meanings and are subject to the provisos specified in claim 1.

12. A bisphosphite-metal complex according to any one of claims 7 to 11, **characterized in that** Q is a hydrocarbon radical of the formula VIII 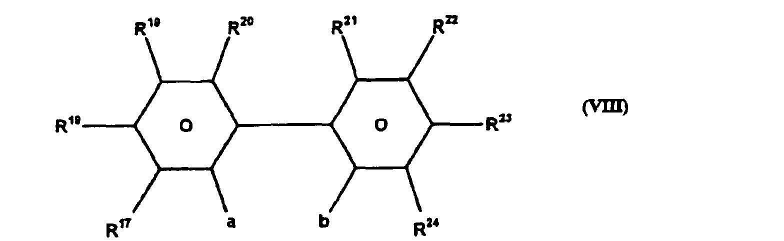 where R¹⁷, R¹⁸, R¹⁹, R²⁰, R²¹, R²², R²³, R²⁴ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, F, Cl, Br, I, -CF₃, -OR²⁵, -COR²⁵, -CO₂R²⁵, -CO₂M, -SR²⁵, -SO₂R²⁵, -SOR²⁵, -SO₃R²⁵, -SO₃M, -SO₂NR²⁵R²⁶, NR²⁵R²⁶, N=CR²⁵R²⁶, NH₂, where R¹⁷ to R²⁴ are identical or different and may be covalently linked to one another,
R²⁵, R²⁶ = H, a substituted or unsubstituted, aliphatic or aromatic hydrocarbon radical having from 1 to 25 carbon atoms,
M = an alkali metal, alkaline earth metal, ammonium or phosphonium ion,
where the positions a and b are linkage points.

13. A bisphosphite-metal complex according to any one of claims 7 to 12, **characterized in that** the metal used is rhodium, platinum, cobalt or ruthenium.

14. The use of a bisphosphite according to any one of claims 1 to 6 in a process for the hydroformylation of olefins.

15. The use of a bisphosphite-metal complex according to any one of claims 7 to 12 in a process for the hydroformylation of olefins.

16. The use of a bisphosphite according to any one of claims 1 to 6 in a process for the hydroformylation of olefins in the presence of further phosphorus-containing ligands.

17. The use of a bisphosphite-metal complex according to any one of claims 7 to 12 in a process for the hydroformylation of olefins in the presence of further phosphorus-containing ligands.

## Revendications

1. Bisphosphite de formule 1 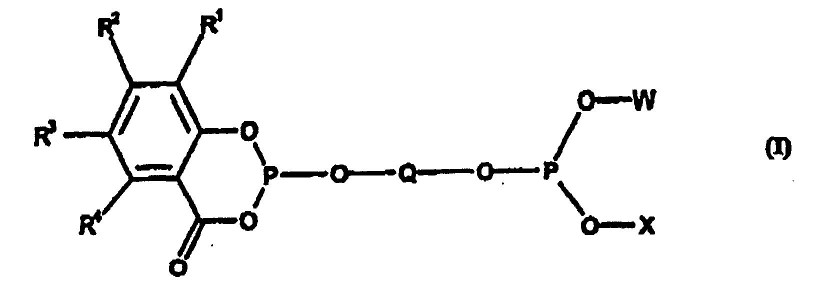 avec R¹, R², R³, R⁴ = H, ou un reste hydrocarboné aliphatique, alicyclique, aliphatico-alicyclique, hétérocycliques, aliphatico-hétérocyclique, aromatique, aromatico-aromatique, ou aliphatico-aromatique, ayant de 1 à 50 atomes de carbone, F, Cl, Br, l, -CF₃, -OR⁷, -COR⁷, -CO₂R⁷,-CO₂M, -SR⁷, -SO₂R⁷, -SOR⁷, -SO₃R⁷, -SO₃M, -SO₂NR⁷R⁸, NR⁷R⁸, N=CR⁷R⁸, NH₂, parmi lesquels R¹ à R⁴ possèdent une signification identique ou différente et peuvent être couplés les uns avec les autres d'une manière covalente R⁷, R⁸ = H, un reste hydrocarboné substitué ou non substitué, aliphatique ou aromatique, ayant de 1 à 25 atomes de carbone, possédant la même signification ou une signification différente,
M = ion de métal alcalin, de métal alcalino-terreux, ammonium, phosphonium,
Q = reste hydrocarboné divalent, aliphatique, alicyclique, aliphatico-alicyclique, hétérocyclique, aliphatico-hétérocyclique, aromatique, aliphatico-aromatiquc, ayant de 1 à 50 atomes de carbone,
W, X = des restes hydrocarbonés aliphatiques, alicycliques, aliphatico-alicycliques, hétérocycliques, aliphatico-hétérocycliques, aromatiques, aromatico-aromatiques, aliphatico-aromatiques, ayant de 1 à 50 atomes de carbone, qui sont identiques ou différents ou peuvent être couplés d'une manière covalente les uns avec les autres.

2. Bisphosphite des formules II, III et IV 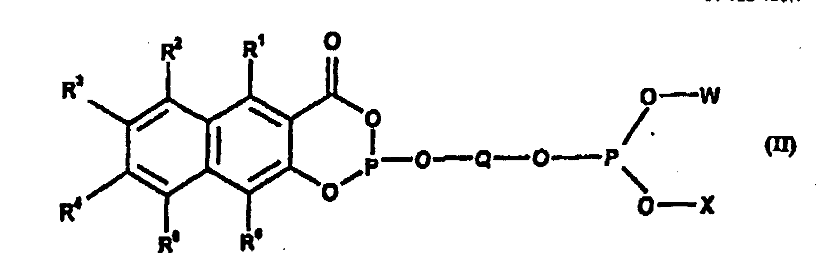 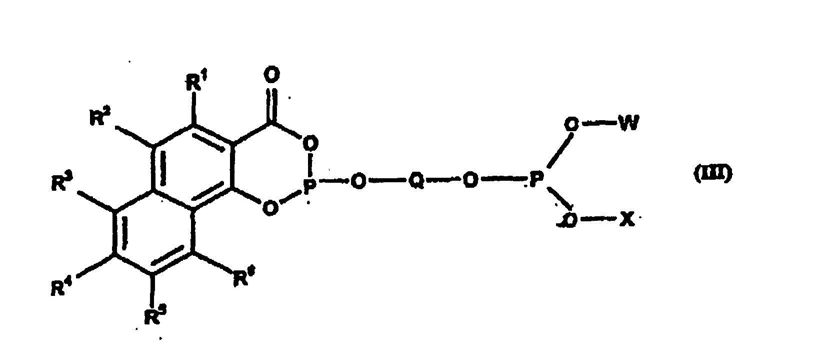 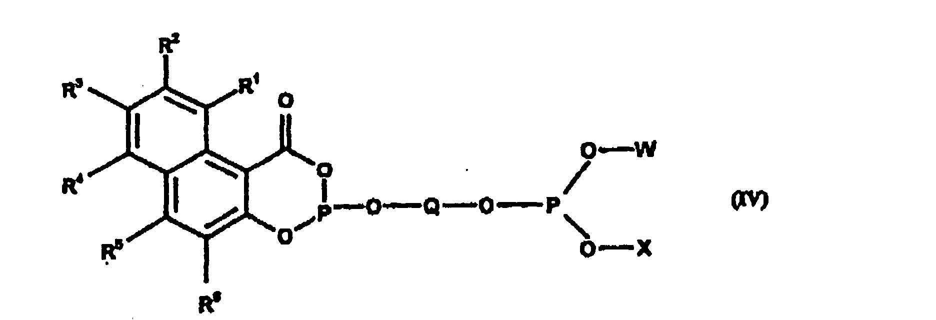 avec R¹, R², R³, R⁴, R⁵, R⁶ = H, reste hydrocarboné aliphatique, alicyclique, aliphatico-alicyclique, hétérocyclique, aliphatico-hétérocyclique, , aromatico-aromatique, aromatique, aliphatico-aromatique, ayant de 1 à 50 atomes de carbone, F, Cl, Br, 1, -CF₃, -OR⁷, -COR⁷, -CO₂R⁷, -CO₂M,-SR⁷, -SO₂R⁷, -SOR⁷, -SO₃R⁷, -SO₃M, -SO₂NR⁷R⁸, NR⁷R⁸, N=CR⁷R⁸, NH₂, parmi lesquels R⁷, R⁸ = H, reste hydrocarboné substitué ou non substitué, aliphatique ou aromatique ayant de 1 à 25 atomes de carbone, possédant une signification identique ou différente,
M = ion de métal alcalin, de métal alcalino-terreux, ion ammonium, ion phosphonium,
Q = reste hydrocarboné divalent, aliphatique, alicyclique, aliphatico-alicyclique, hétérocyclique, aliphatico-hétérocyclique, aromatique, aliphatico-aromatique, ayant de 1 à 50 atomes de carbone,
W, X = des restes hydrocarbonés aliphatiques, alicycliques, aliphatico-alicycliques, hétérocycliques, aliphatico-hétérocycliques, aromatiques, aromatico-aromatiques, aliphatico-aromatiques, ayant de 1 à 50 atomes de carbone, qui peuvent être identiques ou différents ou être couplés d'une manière covalente les uns avec les autres.

3. Bisphophite conformément à l'une quelconque des revendications 1
ou 2,
**caractérisé en ce que**
W et X sont des restes hydrocarbonés aliphatiques, alicycliques, aliphatico-alicycliques, hétérocycliques, aliphatico-hétérocycliques, aromatiques, aromatico-aromatiques, aliphatico-aromatiques, ayant de 1 à 50 atomes de carbone, possédant un couplage covalent conformément à la formule V. 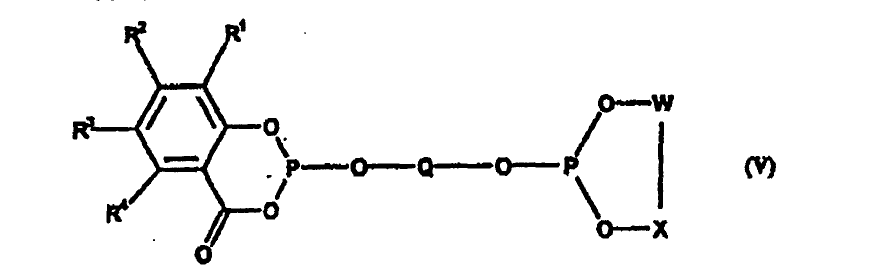 et, R¹, R², R³, R⁴ et Q possèdent les significations et les précisions fournies à la revendication 1.

4. Bisphosphite conformément à l'une quelconque des revendications 1 ou 2,
**caractérisé en ce que**
W et X sont des restes hydrocarbonés aromatiques ayant de 1 à 50 atomes de carbone, possédant les couplages covalents conformément à la formule VI 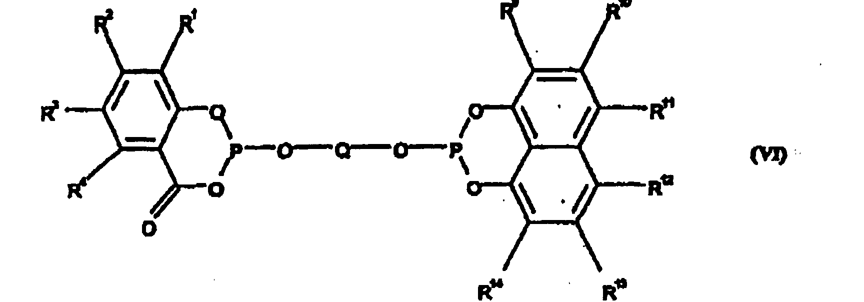 avec R⁹, R¹⁰, R¹¹, R¹², R¹³, R14 = H, un reste hydrocarboné aliphatique, alicycliques, aliphatico-alicycliques, hétérocycliques, aliphatico-hétérocycliques, aromatiques, aromatico-aromatiques, aliphatico-aromatiques, ayant de 1 à 50 atomes de carbone, F, Cl, Br, I, -CF₃,-OR²⁵, -COR²⁵, -CO₂R²⁵, -CO₂M, -SR²⁵, -SO₂R²⁵, -SOR²⁵, -SO₃R²⁵, -SO₃M, - SO₂NR²⁵R²⁶, NR²⁵R²⁶, N=CR²⁵R²⁶, NH₂, parmi lesquels R⁹, R¹⁴, possèdent une signification identique ou différente et peuvent être couplés les uns avec les autres d'une manière covalente.
R²⁵, R²⁶ = H, reste hydrocarboné substitué ou non substitué, aliphatique ou aromatique ayant de 1 à 25 atomes de carbone, possédant la même définition ou une définition différente, M = ion de métal alcalin, alcalino-terreux, ammonium, phosphonium, et
R¹, R², R³, R⁴ et Q possèdent les significations et les précisions fournies à la revendication 1.

5. Bisphosphite conformément à l'une quelconque des revendications 1 ou 2,
**caractérisé en ce que**
W et X sont des restes hydrocarbonés aromatiques ayant de 1 à 50 atomes de carbone possédant un couplage covalent de formule VII, 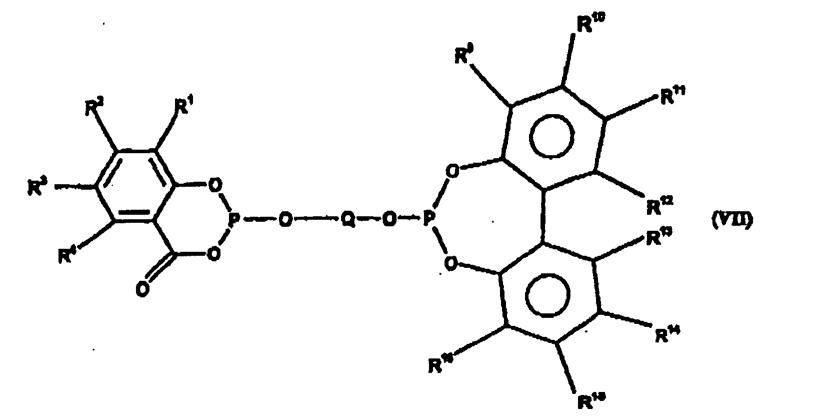 avec R⁹, R¹⁰, R¹¹, R¹², R¹³, R¹⁴, R¹⁵, R¹⁶ = H, un reste hydrocarboné aliphatique, alicycliques, aliphatico-alicycliques, hétérocycliques, aliphatico-hétérocycliques, aromatiques, aromatico-aromatiques, aliphatico-aromatiques, ayant de 1 à 50 atomes de carbone, F, Cl, Br, I, -CF₃,-OR²⁵, -COR²⁵, -CO₂R²⁵, -CO₂M, -SR²⁵, -SO₂R²⁵, -SOR²⁵, -SO₃R²⁵, -SO₃M, - SO₂NR²⁵R²⁶, NR²⁵R²⁶, N=CR²⁵R²⁶, NH₂, parmi lesquels R⁹, R¹⁶, possèdent une signification identique ou différente et peuvent être couplés d'une manière covalente les uns avec les autres,
R²⁵, R²⁶, est de l'hydrogène, un reste hydrocarboné substitué ou non substitué, aliphatique ou aromatique ayant de 1 à 25 atomes de carbone, possédant la même définition ou une définition différente,
M = ion de métal alcalin, alcalino-terreux, ammonium, phosphonium, et R¹, R², R³, R⁴ et Q possèdent les significations et les précisions fournies à la revendication 1.

6. Bisphosphite selon l'une quelconque des revendications 1 à 5,
**caractérisé en ce que**
Q est un reste hydrocarboné conformément à la formule VIII, 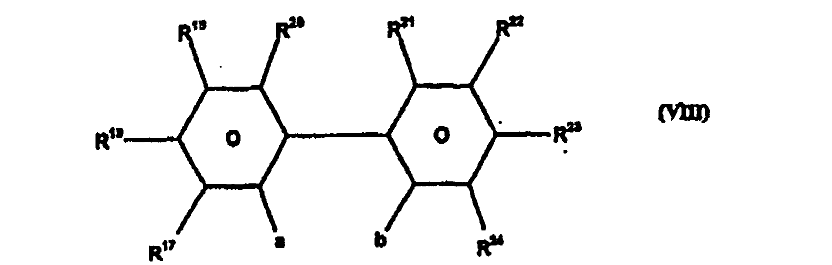 avec R¹⁷, R¹⁸, R¹⁹, R²⁰, R²¹, R²², R²³ = H, un reste hydrocarboné aliphatique, alicycliques, aliphatico-alicycliques, hétérocycliques, aliphatico-hétérocycliques, aromatiques, aromatico-aromatiques, aliphatico-aromatiques, ayant de 1 à 50 atomes de carbone, F, Cl, Br, I, -CF₃,-OR²⁵, -COR²⁵, -CO₂R²⁵, -CO₂M, -SR²⁵, -SO₂R²⁵, -SOR²⁵, -SO₃R²⁵, -SO₃M, - SO₂NR²⁵R²⁶, NR²⁵R²⁶, N=CR²⁵R²⁶, NH₂, pour lesquels R¹⁷, R²⁴, possèdent une signification identique ou différente et peuvent être couplés d'une manière covalente les uns avec les autre,
R²⁵, R²⁶, est de l'hydrogène, un reste hydrocarboné substitué ou non substitué, aliphatique ou aromatique ayant de 1 à 25 atomes de carbone,
M = ion de métal alcalin, alcalino-terreux, ammonium, phosphonium, pour lesquels les positions de a et de b servent de points d'insertion.

7. Complexe métallique à base de bisphophite contenant un métal des 4ème, 5ème, 6ème, 7ème, ou 8ème sous-groupe du système périodique et un ou plusieurs bisphosphites de formule 1, 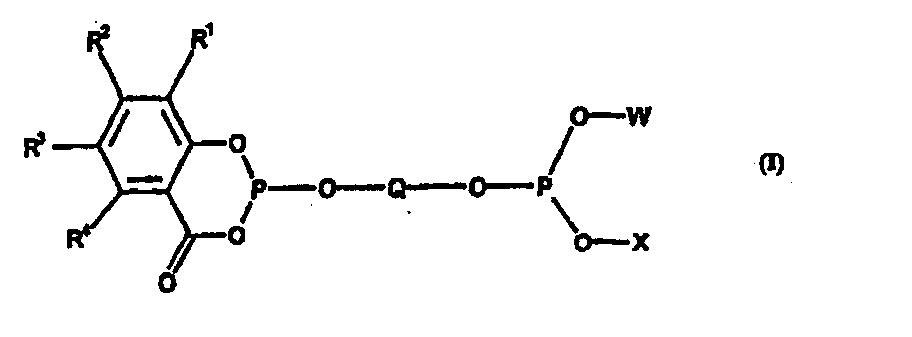 dans laquelle R¹, R², R³, R⁴ = H, un reste hydrocarboné aliphatique, alicyclique, aliphatico-alicyclique, hétérocycliques, aliphatico-hétérocyclique, aromatique, aromatico-aromatique, ou aliphatico-aromatique, ayant de 1 à 50 atomes de carbone, F, Cl, Br, I, -CF₃, -OR⁷, -COR⁷, -CO₂R⁷, -CO₂M, -SR⁷, -SO₂R⁷, -SOR⁷, -SO₃R⁷, -SO₃M,-SO₂NR⁷R⁸, NR⁷R⁸, N=CR⁷R⁸, NH₂, pour lesquels R¹ à R⁴ possèdent une signification identique ou différente et peuvent être couplés les uns avec les autres d'une manière covalente,
R⁷, R⁸ = H, un reste hydrocarboné substitué ou non substitué, aliphatique ou aromatique, ayant de 1 à 25 atomes de carbone, possédant la même signification ou une signification différente,
M = ion de métal alcalin, de métal alcalino-terreux, ammonium, phosphonium,
Q = un reste hydrocarboné, aliphatique, alicyclique, aliphatico-alicyclique, hétérocyclique, aliphatico-hétérocyclique, aromatique, aliphatico-aromatique, ayant de 1 à 50 atomes de carbone,
W, X, sont des restes hydrocarbonés aliphatiques, alicycliques, aliphatico-alicycliques, hétérocycliques, aliphatico-hétérocycliques, aromatiques, aromatico-aromatiques, aliphatico-aromatiques, ayant de 1 à 50 atomes de carbone, qui peuvent être identiques ou différents ou être couplés les uns avec les autres d'une manière covalente.

8. Complexe métallique à base de bisphosphite contenant un métal du 4ème, 5ème, 6ème, 7ème ou 8ème sous-groupe du système périodique des éléments et un ou plusieurs bisphosphites des formules II, III et/ou IV, 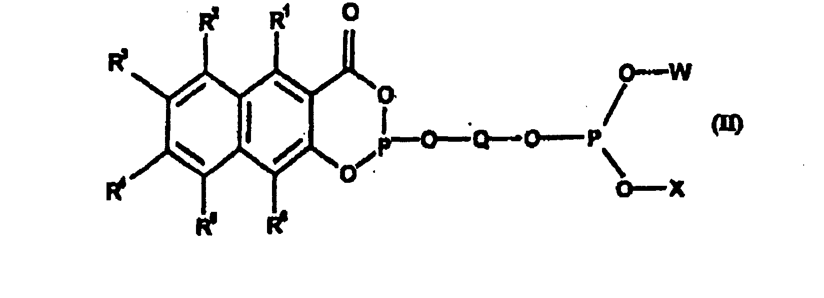 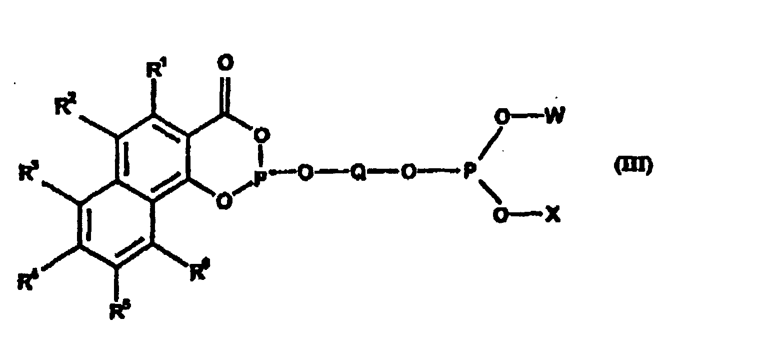 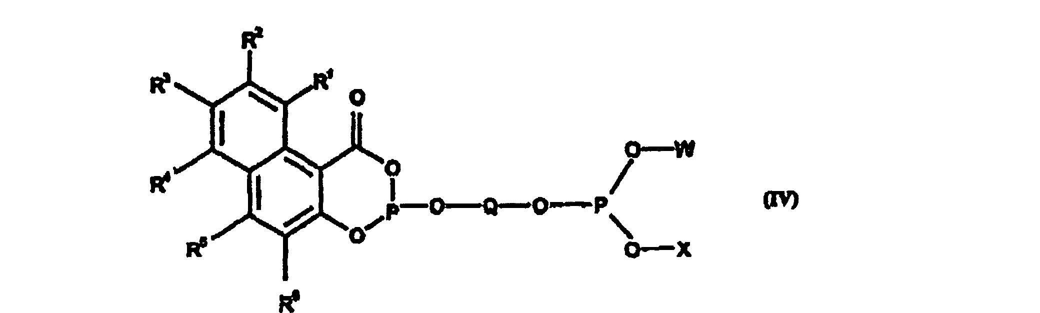 avec R¹, R², R³, R⁴, R⁵, R⁶ = H, un reste hydrocarbonê aliphatique, alicyclique, aliphatico-alicyclique, hétérocycliques, aliphatico-hétérocyclique, aromatique, aromatico-aromatique, ou aliphatico-aromatique, ayant de 1 à 50 atomes de carbone, F, CI, Br, I, -CF₃, -OR⁷, - COR⁷, -CO₂R⁷, -CO₂M, -SR⁷, -SO₂R⁷, -SOR⁷, -SO₃R⁷, -SO₃M,-SO₂NR⁷R⁸, NR⁷R⁸, N=CR⁷R⁸, NH₂, pour lesquels R¹ à R⁷ et R⁸ possèdent une signification identique ou différente,
R⁷, R⁸ son égaux à hydrogène, un reste hydrocarboné substitué ou non substitué, aliphatique ou aromatique, ayant de 1 à 25 atomes de carbone, possédant la même signification ou une signification différente, M est égal à un ion de métal alcalin, de métal alcalino-terreux, ammonium, phosphonium,
Q est égal à un reste hydrocarboné divalent, aliphatique, alicyclique, aliphatico-alicyclique, hétérocyclique, aliphatico-hétérocyclique, aromatique, aliphatico-aromatique, ayant de 1 à 50 atomes de carbone, W, X, sont des restes hydrocarbonés aliphatiques, alicycliques, aliphatico-alicycliques, hétérocycliques, aliphatico-hétérocycliques, aromatiques, aromatico-aromatiques, aliphatico-aromatiques, ayant de 1 à 50 atomes de carbone, qui peuvent être identiques ou différents ou être couplés les uns avec les autres d'une manière covalente

9. Complexe métallique à base de bisphosphite, selon l'une quelconque des revendications 7 ou 8,
**caractérisé en ce que**
W et X sont des restes hydrocarbonés aliphatiques, alicycliques, aliphatico-alicycliques, hétérocycliques, aliphatico-hétérocycliques, aromatiques, aromatico-aromatiques, aliphatico-aromatiques, ayant de 1 à 50 atomes de carbone, possédant un couplage covalent conformément à la formule V, 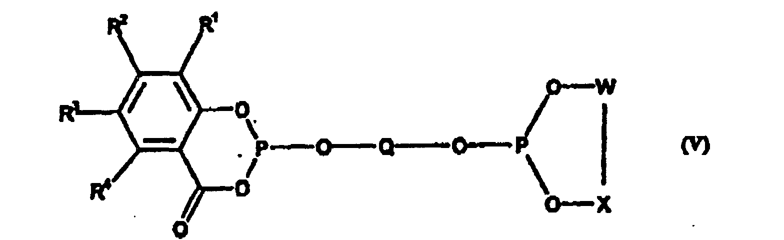 et R¹, R², R³, R⁴ et Q possèdent les significations et les précisions mentionnées à la revendication 7.

10. Complexe métallique à base de bisphosphite, selon l'une quelconque des revendications 7 ou 8,
**caractérisé en ce que**
W et X sont des restes hydrocarbonés aliphatiques, alicycliques, aliphatico-alicycliques, hétérocycliques, aliphatico-hétérocycliques, aromatiques, aromatico-aromatiques, aliphatico-aromatiques, ayant de 1 à 50 atomes de carbone, possédant des couplages covalents conformément à la formule VI. 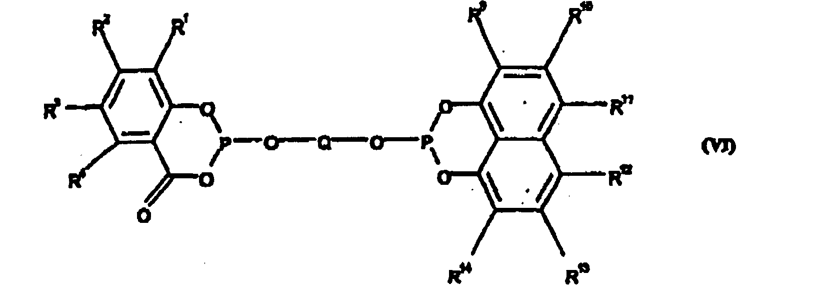 avec R⁹, R¹⁰, R¹¹, R¹², R¹³, R¹⁴ égal à H, un reste hydrocarboné aliphatique, alicycliques, aliphatico-alicycliques, hétérocycliques, aliphatico-hétérocycliques, aromatiques, aromatico-aromatiques, aliphatico-aromatiques, ayant de 1 à 50 atomes de carbonc, F, Cl, Br, I, -CF₃,-OR²⁵, -COR²⁵, -CO₂R²⁵, -CO₂M, -SR²⁵, -SO₂R²⁵, -SOR²⁵, -SO₃R²⁵, -SO₃M, - SO₂NR²⁵R²⁶, NR²⁵R²⁶, N=CR²⁵R²⁶, NH₂, pour lesquels R⁹, R¹⁴, possèdent une signification identique ou différente et peuvent être couplés les uns avec les autres d'une manière covalente.
R²⁵, R²⁶ égaux à H, un reste hydrocarboné substitué ou non substitué, aliphatique ou aromatique ayant de 1 à 25 atomes de carbone, possédant la mème définition ou une définition différente, M = ion de métal alcalin, alcalino-terreux, ammonium, phosphonium, et
R¹, R², R³, R⁴ et Q possèdent les significations et les précisions fournies à la revendication 1.

11. Complexe métallique à base de bisphosphite selon l'une quelconque des revendications 7 ou 8,
**caractérisé en ce que**
W et X sont des restes hydrocarbonés aromatiques ayant de 1 à 50 atomes de carbone possédant un couplage covalent conformément à la formule VII, 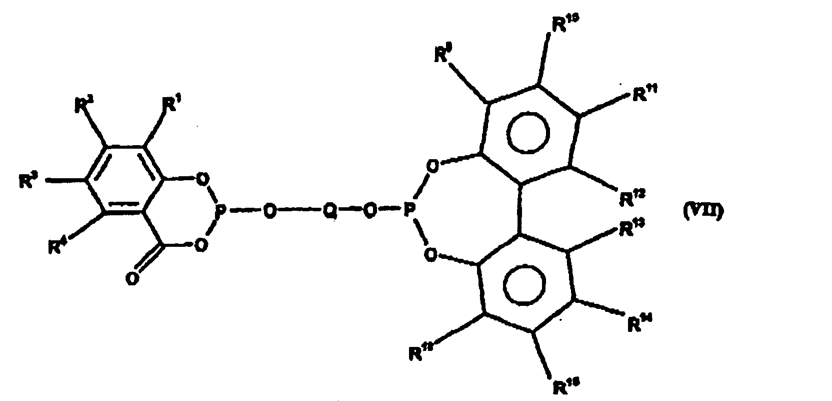 avec R⁹, R¹⁰, R¹¹, R¹², R¹³, R¹⁴, R¹⁵, R¹⁶ égal à H, un reste hydrocarboné aliphatique, alicycliques, aliphatico-alicycliques, hétérocycliques, aliphatico-hétérocycliques, aromatiques, aromatico-aromatiques, aliphatico-aromatiques, ayant de 1 à 50 atomes de carbone, F, Cl, Br, I, -CF₃,-OR²⁵, -COR²⁵, -CO₂R²⁵, -CO₂M, -SR²⁵, -SO₂R²⁵, -SOR²⁵, -SO₃R²⁵, -SO₃M, - SO₂NR²⁵R²⁶, NR²⁵R²⁶, N=CR²⁵R²⁶, NH₂, pour lesquels R⁹, R¹⁶, possèdent une signification identique ou différente et peuvent être couplés les uns avec les autres d'une manière covalente,
R²⁵, R²⁶, sont égaux à H, un reste hydrocarboné substitué ou non substitué, aliphatique ou aromatique ayant de 1 à 25 atomes de carbanc, possédant la même définition ou une définition différente,
M = ion de métal alcalin, alcalino-terreux, ammonium, phosphonium, et
R¹, R², R³, R⁴ et Q possèdent les significations et les précisions fournies à la revendication 1.

12. Complexe métallique à base de bisphosphite selon l'une quelconque des revendications 7 à 11,
**caractérisé en ce que**
Q est un reste hydrocarboné conformément à la formule VIII, 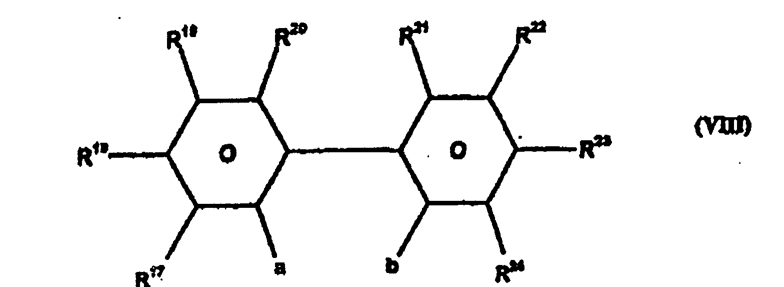 avec R¹⁷, R¹⁸, R¹⁹, R²⁰, R²¹, R²², R²³ = H, un reste hydrocarboné aliphatique, alicycliques, aliphatico-alicycliques, hétérocycliques, aliphatico-hétérocycliques, aromatiques, aromatico-aromatiques, aliphatico-aromatiques, ayant de 1 à 50 atomes de carbone, F, Cl, Br, I, -CF₃,-OR²⁵, -COR²⁵, -CO₂R²⁵, -CO₂M, -SR²⁵, -SO₂R²⁵, -SOR²⁵, -SO₃R²⁵, -SO₃M, - SO₂NR²⁵R²⁶, NR²⁵R²⁶, N=CR²⁵R²⁶, NH₂, pour lesquels R¹⁷, R²⁴, possèdent une signification identique ou différente et peuvent être couplés les uns avec les autres d'une manière covalente,
R²⁵, R²⁶, sont égaux à H, un reste hydrocarboné substitué ou non substitué, aliphatique ou aromatique ayant de 1 à 25 atomes de carbone,
M est égal à un ion de métal alcalin, alcalino-terreux, ammonium, phosphonium, pour lesquels les positions de a et b servent comme point d'insertion.

13. Complexe métallique à base de bisphosphite selon l'une quelconque des revendications 7 à 12,
**caractérisé en ce que**
comme métal on met en oeuvre du rhodium, du platine, du cobalt, ou du ruthénium.

14. Utilisation des bisphosphites conformément à l'une quelconque des revendications 1 à 6, dans un procédé d'hydroformylation d'oléfines.

15. Utilisation des complexes métalliques de bisphosphite conformément à l'une quelconque des revendications 7 à 12, dans un procédé d'hydroformylation d'oléfines.

16. Utilisation de bisphosphite conformément à l'une quelconque des revendications 1 à 6, dans un procédé d'hydroformylation d'oléfines en présence d'autres ligands phosphorés.

17. Utilisation de complexes métalliques à base de bisphosphite conformément à l'une quelconque des revendications 7 à 12, dans un procédé d'hydroformylation d'oléfines en présence d'autres ligands phosphorés.
